# EUROPEAN PATENT APPLICATION

(11) **EP 4 074 824 A1**
(43) Date of publication of application: **19.10.2022**
(21) Application number: 22155855.4
(22) Date of filing: 07.08.2017
(51) Int. Cl.: C12N 15/10, C12Q 1/6848

(54) **REDUCTION OF SIGNAL FROM CONTAMINANT NUCLEIC ACIDS**

(30) Priority: 08.08.2016 US 201662372269 P
(62) Divisional of application: 17840106.3
(71) Applicant: Karius, Inc., Redwood City, CA 94065 (US)
(72) Inventor: BLAUWKAMP, Timothy, A., Palo Alto, CA 94303 (US); CHRISTIANS, Fred, Los Altos Hill, CA 94022 (US); ROSEN, Michael, Palo Alto, CA 94036 (US); VILFAN, Igor, D., Palo Alto, CA 94303 (US)
(74) Representative: J A Kemp LLP

(57) **Abstract**

**ABSTRACT OF THE DISCLOSURE**

This disclosure provides methods that are useful for reducing or inactivating contaminant nucleic acids.

## Description

### CROSS-REFERENCE

The application claims the benefit of U.S. Provisional Patent Application No. 62/372,269 (Attorney Docket No. 47697-707.101) filed August 08, 2016; the entire contents of which is incorporated herein by reference.

### BACKGROUND OF THE INVENTION

Next generation sequencing can be used to gather massive amounts of data about the genetic content of a sample. It can be particularly useful for analyzing nucleic acids in complex samples, such as clinical samples and for the sequencing of whole genomes. However, next generation sequencing can be so sensitive that it may detect even trace amounts of nucleic acids, such as contaminant nucleic acids.

Contaminant nucleic acids may be introduced during sample collection, processing or manipulation, particularly when the sample is exposed to microbes present in a laboratory or clinical environment. Contaminant nucleic acids may be confused with nucleic acids in the original sample, which may lead to false positive results and negative clinical outcomes. There is thus an urgent need in the art for methods of reducing the signal generated from contaminant nucleic acids in sequencing and other assays.

### SUMMARY OF THE INVENTION

The present disclosure generally provides methods for improved next generation sequencing of clinical samples containing contaminating nucleic acids that resemble the sequence, concentration, or fragment length of target nucleic acids. In some examples, the clinical sample is derived from a patient suspected of being infected with a particular pathogen, and the methods provided herein remove or reduce signal from contaminating nucleic acids derived from environmental pathogens, thereby improving the accuracy and efficiency of the sequencing assay.

The present disclosure generally provides methods for reducing or inactivating contaminant nucleic acids in biological reagents (e.g., buffers, solutions, kits, blood storage reagents ) or labware (e.g., pipette tips, pipettes, syringes, plates, test tubes, glassware, vials, columns, instrumentation) in order to prepare samples for assays such as sequencing assays. Often, the methods involve contacting a reagent (e.g., nucleic acid extraction reagent) with an inactivating agent that ultimately may reduce or eliminate signal from contaminant nucleic acids. In some cases, the methods provided herein may be used for inactivating contaminant nucleic acids in a sequencing library by cleaning up the reagents and/or labware used during sample collection, sample processing, and library production.

Provided herein is a method for inactivating contaminant nucleic acids, the method comprising: (a) heating a nucleic acid analysis buffer, wherein the nucleic acid analysis buffer comprises contaminant nucleic acids and wherein the heating lasts for a length of time sufficient to inactivate at least a portion of the contaminant nucleic acids, thereby producing a decontaminated nucleic acid analysis buffer; and (b) contacting the decontaminated nucleic acid analysis buffer of step (a) with a sample comprising target nucleic acids.

Provided herein is a method of conducting a nucleic acid assay, the method comprising: (a) exposing a nucleic acid analysis buffer to non-ionizing radiation or to a nuclease, wherein the nucleic acid analysis buffer comprises contaminant nucleic acids and wherein the exposure to the non-ionizing radiation or to the nuclease lasts for a length of time sufficient to inactivate at least a portion of the contaminant nucleic acids, thereby producing a decontaminated nucleic acid analysis buffer; (b) contacting the decontaminated nucleic acid analysis buffer of step (a) with a sample comprising target nucleic acids; and (c) contacting the target nucleic acids in the sample with amplification or sequencing reagents, wherein the amplification or sequencing reagents have not been subjected to endonuclease treatment to remove contaminant nucleic acids.

Provided herein is a method of conducting a sequencing assay, the method comprising: (a) heating a nucleic acid analysis buffer, wherein the nucleic acid analysis buffer comprises contaminant nucleic acids and wherein the heating lasts for a length of time sufficient to inactivate at least a portion of the contaminant nucleic acids, thereby producing a decontaminated nucleic acid analysis buffer; contacting the decontaminated nucleic acid analysis buffer of step (a) with a sample comprising target nucleic acids; attaching double stranded adapters to the target nucleic acids in order to produce tagged target nucleic acids; and subjecting the tagged target nucleic acids to a sequencing assay. In one embodiment, the method comprises performing an amplification reaction after attaching the double stranded adapters to the target nucleic acids.

In some embodiments, the contaminant nucleic acids are double-stranded nucleic acids. In some embodiments, the contaminant nucleic acids are single stranded nucleic acids. In some embodiments, the contaminant nucleic acids comprise RNA or DNA. In some embodiments, the contaminant nucleic acids are less than 75 nucleotides in length. In some embodiments, the contaminant nucleic acids are greater than 1,000 nucleotides in length. In some embodiments, the contaminant nucleic acids are a mixture of nucleic acids having different lengths, wherein a portion of the contaminant nucleic acids contains short nucleic acids less than 75 nucleotides in length and a portion of the contaminant nucleic acids contains long nucleic acids greater than 500 nucleotides in length. In some embodiments, the short nucleic acids are inactivated by applying heat to the nucleic acid analysis buffer and the long nucleic acids are inactivated by applying non-ionizing radiation to the nucleic acid analysis buffer.

In some embodiments, the target nucleic acids are double-stranded nucleic acids. In some embodiments, the double-stranded target nucleic acids are natural double-stranded nucleic acids present in the sample prior to any sample processing. In some embodiments, the double-stranded target nucleic acids are double-stranded as a result of an *in vitro* amplification reaction. In some embodiments, the target nucleic acids comprise RNA or DNA.

Provided herein is a method for inactivating contaminant nucleic acids in a buffer, the method comprising: (a) obtaining a nucleic acid analysis buffer, wherein the nucleic acid analysis buffer comprises: (i) a reagent that is not water and (ii) contaminant nucleic acids; and (b) exposing the nucleic acid analysis buffer to ionizing radiation or a nuclease for a length of time sufficient to inactivate at least a portion of the contaminant nucleic acids, thereby producing a decontaminated nucleic acid analysis buffer. The method of any one of the preceding claims, wherein the nucleic acid analysis buffer is rotated or agitated during the heating of the nucleic acid analysis buffer.

In some cases of a method described herein, the contaminant nucleic acids are derived from bacteria. In some cases of a method described herein, the contaminant nucleic acids comprise nucleic acids derived from soil bacteria, Propionibacterium acnes, Bradyrhizobium sp. S23321, *Bradyrhizobium diazoefficiens, Bradyrhizobium japonicum,* Acidovorax sp. KKS102, Bradyrhizobium sp. BTAi1, *Acidovorax temperans, Delftia sp. Csl-4, Delftia acidovorans, Methylobacterium acidovorans, Rhodopseudomonas palustris, Serratia marcescens, Acinetobacter junii, Acinetobacter johnsonii, Chryseobacterium indologenes* or any combination thereof. In some cases of a method described herein, the contaminant nucleic acids comprise nucleic acids derived from *Bradyrhizobium, Rhizobium*/*Agrobacterium, Sphingomonas, Burkholderia, Ralstonia, Pseudomonas, Stenotrophomonas, Flavobacterium, Bradyrhizobium sp. DFCI-1, Escherichia coli, Bos, Sus, Gallus, Herbaspirillum, Methylobacteria* or any combination thereof.

In some cases of a method described herein, the contaminant nucleic acids are present at a concentration of at least 0.1% of total nucleic acids prior to inactivation. In some cases of a method described herein, the contaminant nucleic acids are present at a concentration of up to 0.001% after inactivation. In some cases of a method described herein, a signal from the contaminant nucleic acids is reduced by at least 50%, at least 60%, at least 70%, at least 80%, or between 50%-80%, following the inactivation.

In some cases of a method described herein, inactivating the contaminant nucleic acids prevents the contaminant nucleic acids from being sequenced or PCR amplified. In some cases of a method described herein, inactivating the contaminant nucleic acids denatures the contaminant nucleic acids. In some cases of a method described herein, inactivating the contaminant nucleic acids generates pyrimidine dimers in the contaminant nucleic acids. In some cases of a method described herein, the pyrimidine dimers are selected from cyclobutane pyrimidine dimers, thymine dimers, 6,4-photoproducts, and any combination thereof. In some cases of a method described herein, inactivating the contaminant nucleic acids generates double-strand breaks or single-strand breaks in the contaminant nucleic acids.

In some cases of a method described herein, the nucleic acid analysis buffer is at least one buffer selected from the group consisting of a nucleic acid elution buffer, nucleic acid binding buffer, nucleic acid wash buffer, nucleic acid extraction buffer, hybridization reagent, equilibration buffer, purification reagent, sample preparation reagent, deparaffinization solution, cell lysis buffer, sample lysis buffer, tissue lysis buffer, viral particle lysis buffer, suspension or resuspension buffer, neutralization buffer, digestion buffer, equilibration buffer, solubilization buffer, blood/plasma stabilization reagent, blood component separation gel, and a PCR buffer. In some embodiments, the nucleic acid binding buffer comprises nucleic acid binding particles. In some embodiments, the nucleic acid binding particles comprise magnetic particles. In some cases of a method described herein, the nucleic acid analysis buffer is an extraction buffer. In some embodiments, the method comprises extracting the target nucleic acids following the contacting of the nucleic acid analysis buffer with the sample comprising target nucleic acids.

In some cases of a method described herein, the nucleic acid analysis buffer comprises a detergent, a surfactant, a salt, a sugar, a protein, an alcohol, or a stabilizer. In some cases of a method described herein, the nucleic acid analysis buffer further comprises water. In some cases of a method described herein, the nucleic acid analysis buffer comprises at least one detergent. In some cases of a method described herein, the nucleic acid analysis buffer comprises a sugar such as glycerol. In some embodiments, the nucleic acid analysis buffer is not a polymerase chain reaction (PCR) buffer or a ligase buffer.

In some cases of a method described herein, the method further comprises amplifying a portion of the target nucleic acids in the sample with amplification reagents. In some cases, the amplification reagents include one or more of the following: polymerase, reverse transcriptase, primers, TaqMan probe, dNTPs (e.g., dATP, dCTP, dGTP, dTTP), or MgCl₂.

In some cases of a method described herein, the nucleic acid analysis buffer retains at least 50% of its activity following the heating. In some cases of a method described herein, the nucleic acid analysis buffer retains at least 50% of its activity following the exposure to the non-ionizing radiation or the nuclease. In some cases of a method described herein, the nucleic acid analysis buffer retains at least 50% of its activity following the exposure to the ionizing radiation or the nuclease.

In some cases of a method described herein, the method further comprises sequencing at least a portion of the target nucleic acids. In some cases of a method described herein, the sequencing comprises next generation sequencing. In some cases of a method described herein, the method further comprises amplifying at least a portion of the target nucleic acids.

In some cases of a method described herein, the method further comprises heating the nucleic acid analysis buffer prior to the contacting the decontaminated nucleic acid analysis buffer with a sample comprising nucleic acids. In some cases of a method described herein, the method further comprises heating the nucleic acid analysis buffer. In some embodiments, the method further comprises subjecting the nucleic acid analysis buffer to non-ionizing radiation.

In some cases of a method described herein, the method further comprises cooling the nucleic acid analysis buffer following the heating. In some cases of a method described herein, the nucleic acid analysis buffer is cooled to room temperature. In some cases of a method described herein, the nucleic acid analysis buffer is cooled for a period less than 30 seconds, less than 20 seconds, less than 10 seconds, less than 5 seconds, less than 3 seconds, or less than 1 second. In some cases of a method described herein, the nucleic acid analysis buffer is cooled to a temperature less than 80 °C in less than 30 seconds, less than 20 seconds, less than 10 seconds, less than 5 seconds, less than 3 seconds, or less than 1 second. In some cases of a method described herein, the nucleic acid analysis buffer is cooled to a temperature less than 60 °C in less than 30 seconds, less than 20 seconds, less than 10 seconds, less than 5 seconds, less than 3 seconds, or less than 1 second. In some cases of a method described herein, the nucleic acid analysis buffer is cooled to a temperature less than 40 °C in less than 30 seconds, less than 20 seconds, less than 10 seconds, less than 5 seconds, less than 3 seconds, or less than 1 second.

In some cases of a method described herein, the ionizing radiation is gamma radiation. In some cases of a method described herein, the non-ionizing radiation is ultraviolet radiation.

In some cases of a method described herein, the heating occurs at a temperature of at least about 80 °C. In some embodiments, the heating the nucleic acid buffer is performed for at least 30 minutes. In some cases of a method described herein, the heating occurs for at least 15 seconds.

In some cases of a method described herein, the contacting the decontaminated nucleic acids analysis buffer with the sample comprising nucleic acids occurs within 30 minutes following step (a). In some cases of a method described herein, the contacting the decontaminated nucleic acids analysis buffer with the sample comprising nucleic acids occurs at least one minute following step (a).

In some cases of a method described herein, the method further comprises irradiating the contaminant nucleic acids. In some cases of a method described herein, the irradiating comprises UV or gamma radiation. In some cases of a method described herein, the irradiating is at a frequency of at least 200 nm. In some cases of a method described herein, the irradiating occurs for at least one minute. In some embodiments, a dose of UV irradiation is at least about 10,000 Joules per meter squared (J/m²). In some embodiments, a dose of gamma irradiation is at least about 15 kiloGray (kGy).

In some cases of a method described herein, the method further comprises contacting the decontaminated nucleic acid analysis buffer with a sample comprising target nucleic acids.

In some cases of a method described herein, the method further comprises amplifying a portion of the target nucleic acids in the sample with amplification reagents.

In some cases of a method described herein, the amplification reagents are not exposed to enzymatic treatment to degrade contaminant nucleic acids.

In some cases of a method described herein, the sample comprising target nucleic acids comprises intact cells. In some cases of a method described herein, the sample comprising target nucleic acids comprises DNA. In some cases of a method described herein, the sample comprising target nucleic acids comprises RNA. In some cases of a method described herein, the target nucleic acids in the sample are derived from blood, plasma, serum, saliva, bronchoalveolar lavage, nasal swab, lymph, cerebrospinal fluid or urine.

In some cases of a method described herein, the target nucleic acids in the sample comprise cell-free nucleic acids originally present as cell-free nucleic acids in a circulating bodily fluid or urine. In some cases of a method described herein, the cell-free nucleic acids are DNA. In some cases of a method described herein, the sample comprises human nucleic acids. In some cases of a method described herein, the human has a pathogenic infection, is at risk of having a pathogenic infection, or is suspected of having a pathogenic infection. In some cases of a method described herein, the pathogenic infection is a bacterial, viral, fungal or parasitic infection. In some cases of a method described herein, the pathogenic infection is a bacterial infection. In some cases of a method described herein, the contaminant nucleic acids are derived from a pathogen. In some cases of a method described herein, the pathogenic infection is a bacterial infection and the contaminant nucleic acids are bacterial nucleic acids.

In some cases of a method described herein, the method further comprises detecting a condition of the sample. In some cases of a method described herein, the condition is a pathogenic infection. In some cases of a method described herein, the nuclease is an endonuclease, RNase, or DNase. In some cases of a method described herein, the nuclease is DNase.

In some cases of a method described herein, the reagent that is not water is a reagent selected from the group consisting of a detergent, a surfactant, a salt, a sugar, a protein, an alcohol, and a stabilizer. In some cases of a method described herein, the nucleic acid analysis buffer further comprises water. In some cases of a method described herein, the nucleic acid analysis buffer comprises at least one detergent. In some cases of a method described herein, the reagent that is not water is a sugar such as glycerol. In some embodiments, the method further comprises attaching an adapter to the target nucleic acids.

### INCORPORATION BY REFERENCE

All publications, patents, and patent applications mentioned in this specification are herein incorporated by reference in their entireties to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**Fig. 1** is a schematic depicting an overview of methods of this disclosure.
**Fig. 2** is a schematic showing exemplary methods for removing or reducing contaminant nucleic acids present in a nucleic acid analysis buffer.
**Fig. 3** shows the effect of cleaning/replacement, UV treatment, and heat treatment on the abundance of microbes when compared to untreated sample.
**Fig. 4** shows the effect of cleaning/replacement (cleaned method), UV treatment, and heat treatment on the abundance of microbes when compared to untreated sample.
**Fig. 5** shows the effect of UV treatment of control nucleic acid samples.

### DETAILED DESCRIPTION OF THE INVENTION

### Overview

This disclosure provides multiple methods and approaches for inactivating nucleic acid contaminants or otherwise reducing or eliminating the signal produced by contaminants in sequencing and other assays. Generally, the methods provided herein involve contacting a reagent used for a nucleic acid assay with an inactivation agent such as non-ionizing radiation, ionizing radiation, or a nuclease enzyme. The methods may involve the complete or substantial inactivation or removal of contaminating nucleic acids from nucleic acid reagents such as extraction buffers, lysis buffers and the like. By reducing or eliminating signal from pathogen contaminants in nucleic acid reagents, the methods may be especially helpful for increasing the accuracy and efficiency of assays designed to detect low-abundance pathogens in clinical samples and reduce the undesirable outcomes that occur when a pathogen contaminant is confused with an actual pathogen present in an original sample.

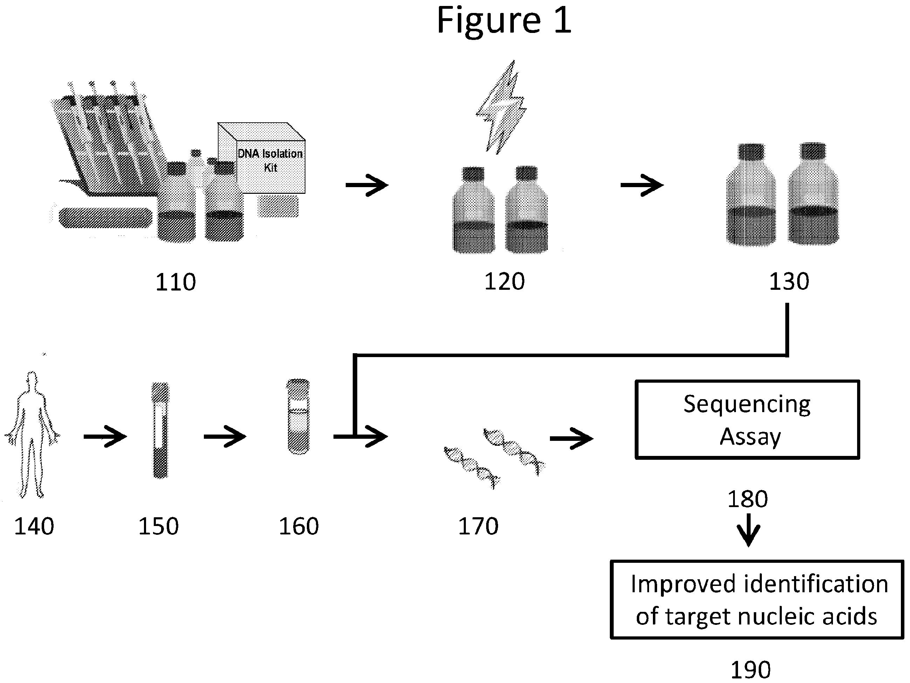

**Fig. 1** provides a general overview of the steps of many of the methods provided herein. The method may involve obtaining nucleic acid reagents **110,** which may contain, or be suspected of containing, contaminant nucleic acids introduced from a laboratory, research, or manufacturing plant environment. The nucleic acid reagents may be any nucleic acid reagents used for sample processing, including lysis buffers, extraction buffers, neutralization buffers, equilibration buffers and the like. Often, the nucleic acid reagents contain detergent and other components for the lysis or extraction reactions. In some instances, the nucleic acid assay reagent contains a detergent, a surfactant, a salt, a sugar, a protein, an alcohol, and/or a stabilizer or other reagent that is not water. Usually, the nucleic acid assay reagent also contains water. The methods provided herein may include an inactivation step **120,** wherein the nucleic acid assay reagents are inactivated in some manner such as by exposure to radiation or heat or both, resulting in a nucleic acid reagent with inactivated contaminant nucleic acids **130.** In some cases, when the inactivation step is by exposure to heat, the step may further comprise cooling the nucleic acid assay reagents to a lower temperature. In some cases, the cooling process is a rapid cooling process. In some cases, the nucleic acid contaminants may be wholly or partially inactivated or removed by the methods provided herein. The inactivation step may cause denaturation of the contaminant nucleic acids, particularly when the inactivation agent is heat. In some cases, the contaminant nucleic acids are inactivated by some other structural alteration or by enzymatic degradation. In some cases, the nucleic acids are inactivated simply by being physically removed from the nucleic acid assay reagent using a filtration device or other device known in the art. The contaminating nucleic acids may also be removed or isolated bioinformatically, such as during analysis of sequencing data. The bioinformatic step may be used as the sole mode of removing data generated by contaminating nucleic acids, or may be used in conjunction with an inactivation step or removal step.

The methods may also involve obtaining a sample from a subject **140,** such as a patient. In some particular embodiments, the subject has an infectious disease or is otherwise suspected of being infected with a pathogen. The sample may be a blood sample **150,** as depicted, or any other type of biological sample, especially a biological sample containing a bodily fluid, tissue, and/or cells.

A nucleic acid assay reagent that is subjected to the inactivation or removal steps described herein may be used in an assay, such as a sequencing assay (e.g., next generation sequencing assay) without introducing significant amounts of contaminating nucleic acids into the analysis. For example, in cases where the nucleic acid assay reagent is an extraction buffer, the buffer may be used to extract nucleic acids from a sample; and the extracted nucleic acids may be used in downstream next generation sequencing analyses. Thus, as shown in step **160,** the nucleic acid assay reagent **130** processed by the methods provided herein may be used in a nucleic acid assay, where it contacts a biological sample, such as the blood sample **150.** In such assay, the nucleic acid assay reagent may carry out its originally-intended purpose, such as nucleic acid extraction, cell lysis, or neutralization. Importantly, the nucleic acid reagent may retain all of, or a substantial degree of, its original activity level even after the inactivation step. The resulting nucleic acids **170** may then be analyzed by a sequencing assay **180** such as a next generation sequencing assay. Since the nucleic acid contaminants are generally either inactivated or removed from the nucleic acid assay reagent via an approach outlined herein, the contaminant nucleic acids are largely undetected by the sequencing assay. As a result, a condition of the subject can be identified with a higher accuracy and level of certainty **190.** In some particular embodiments, the sequencing assay (e.g., next generation sequencing assay) detects pathogen nucleic acids within a sample of cell-free nucleic acids (e.g., DNA) derived from a human patient.

The steps may be performed in any order and in any combination. In some cases, certain steps are repeated several times. In some cases, certain steps are not performed. In some cases, new steps are added to, or interspersed between, the depicted steps.

The methods provided herein enable improved detection of target nucleic acids by next generation sequencing, particularly when contaminants matching the concentration, sequence identity, or fragment length of the target nucleic acids are present in the sample. For example, accurate detection and quantification of target pathogens in clinical samples by next generation sequencing may be undermined or negatively impacted if the samples are contaminated with microbes with sequence identity to the pathogen. The methods provided herein thus may help avoid the pitfalls that arise when a resemblance between target and contaminant is sufficient to cause a false positive result or otherwise cloud analysis of sequencing data.

### Samples

The samples analyzed in the methods provided herein are preferably any type of clinical sample. In some cases, the samples contain cells, tissue, or a bodily fluid. In preferred embodiments, the sample is a liquid or fluid sample. In some cases, the sample contains a body fluid such as whole blood, plasma, serum, urine, saliva, lymph, cerebrospinal fluid, bronchoalveolar lavage, nasal swab, respiratory secretions, vaginal fluid, amniotic fluid, semen or menses. In some cases, the sample is made up of, in whole or in part, cells or tissue. The sample (particularly cellular samples or tissue biopsies) may be from any part of the body including the central nervous system, the brain, spinal cord, bone marrow, pancreas, thyroid, gall bladder, liver, heart, spleen, colon, rectum, lung, respiratory system, throat, nasal cavity, stomach, esophagus, ears, eyes, skin, limbs, uterus, prostate, reproductive organ, or any other organ or region of the body.

Generally, the samples are from a human subject, especially human patients. But the samples may also be from any other type of subject including any mammal, non-human mammal, non-human primate, domesticated animal (e.g., laboratory animals, household pets, or livestock), or non-domesticated animal (e.g., wildlife). In preferred embodiments, the subject is a host organism (e.g., a human) infected with a pathogen, at risk of infection by a pathogen, or suspected of having a pathogenic infection. In some cases, the subject is suspected of having a particular infection, e.g., suspected having tuberculosis. In other cases, the subject is suspected of having an infection of unknown origin. In some particular embodiments, the subject is a dog, cat, rodent, mouse, hamster, cow, bird, chicken, pig, horse, goat, sheep, rabbit, ape, monkey, or chimpanzee.

In some cases, the subject may have been treated or may be treated with an antimicrobial, antibacterial, antiviral, or antiparasitic drug. In some cases, the subject is infected (e.g., with one or more microbes, pathogens, bacteria, viruses, fungi, or parasites). In some cases, the subject is not infected (e.g., with one or more microbes, pathogens, bacteria, viruses, fungi, or parasites). In some cases, the subject is healthy. In some cases, the subject is susceptible or at risk of an infection. The subject may have or be at risk of having another disease or disorder. For example, the subject may have, be at risk of having, or be suspected of having a disease such as cancer (e.g., breast cancer, lung cancer, pancreatic cancer, hematological cancer, etc.).

The sample may be a nucleic acid sample; in some cases, the sample contains a certain amount of nucleic acids. Nucleic acids within a sample may include double-stranded (ds) nucleic acids, single stranded (ss) nucleic acids, DNA, RNA, cDNA, mRNA, cRNA, tRNA, ribosomal RNA, dsDNA, ssDNA, miRNA, siRNA, circulating nucleic acids, circulating cell-free nucleic acids, circulating DNA, circulating RNA, cell-free nucleic acids, cell-free DNA, cell-free RNA, circulating cell-free DNA, circulating cell-free RNA, genomic DNA, exosomes, mitochondrial nucleic acids, circulating tumor DNA, circulating tumor RNA, or any combination thereof. In some cases, sample nucleic acids may include synthetic nucleic acids. In some cases, the sample may be an unprocessed sample (e.g., whole blood) or a processed sample (e.g., serum, plasma) that contains cell-free or cell-associated nucleic acids. In some cases, the sample has been enriched for a certain type of nucleic acid, e.g., DNA, RNA, cell-free DNA, cell-free RNA, cell-free circulating DNA, cell-free circulating RNA, etc. In some cases, a sample has been processed in some way to isolate nucleic acids or to separate nucleic acids from other components within the sample.

Often, the sample is a fresh sample. In some cases, the sample is a frozen sample. In some cases, the sample is fixed, e.g., with a chemical fixative.

### Target Nucleic Acids

The methods provided herein may be used to detect any number of target nucleic acids. The target nucleic acids may include whole or partial genomes, exomes, genetic loci, genes, exons, introns, modified nucleic acids (e.g., methylated nucleic acids), etc. Often, the methods provided herein can be used to detect pathogen target nucleic acids; in some cases, the pathogen target nucleic acids are present in complex clinical sample containing nucleic acids from the subject. The pathogen target nucleic acid may be associated with an infectious disease, such as influenza, tuberculosis, or any other known infectious disease or disorder, including those described further herein.

In some cases, the pathogen target nucleic acid is present in a tissue sample, such as a tissue sample from a site of infection. In other cases, the pathogen target nucleic acid has migrated from the site of infection; for example, it may be obtained from a sample containing circulating cell-free nucleic acids (e.g., DNA).

In some cases, the target nucleic acid derives from cancer tissue. The target nucleic acid may be obtained directly from the tissue or tumor. In some cases, the target cancer nucleic acid is obtained from circulating cell-free nucleic acids or from circulating tumor cells (CTCs).

In some cases, the target nucleic acid may make up only a very small portion of the entire sample, e.g., less than 0.1 %, less than 0.01%, less than 0.001%, less than 0.0001%, less than 0.00001%, less than 0.000001%, less than 0.0000001% of the total nucleic acids in a sample. Often, the total nucleic acids in an original sample may vary. For example, total cell-free nucleic acids (e.g., DNA, mRNA, RNA) may be in a range of 1-100 ng/ml, e.g., (about 1, 5, 10, 20, 30, 40, 50, 80, 100 ng/ml). In some cases, the total concentration of cell-free nucleic acids in a sample is outside of this range (e.g., less than 1 ng/ml; in other cases, the total concentration is greater than 100 ng/ml). This may be the case with cell-free nucleic acid (e.g., DNA) samples that are predominantly made up of human DNA and/or RNA. In such samples, pathogen target nucleic acids or cancer target nucleic acids may have scant presence compared to the human or healthy nucleic acids.

The length of target nucleic acids can vary. In some cases, target nucleic acids may be about or at least about 20, 30, 40, 50, 60, 70, 80, 90, 100, 110, 120, 130, 140, 150, 160, 170, 180, 190, 200, 250, 300, 350, 400, 450, 500, 750, 1000, 1500 or 2000 nucleotides (or base pairs) in length. In some cases, target nucleic acids may be up to about 20, 30, 40, 50, 60, 70, 80, 90, 100, 110, 120, 130, 140, 150, 160, 170, 180, 190, 200, 250, 300, 350, 400, 450, 500, 750, 1000, 1500 or 2000 nucleotides (or base pairs) in length. In some particular embodiments, the target nucleic acids are relatively short, e.g., less than 500 base pairs (or nucleotides) or less than 1000 base pairs (or nucleotides) in length. In some cases, the target nucleic acids are relatively long, e.g., greater than 1000, greater than 1500, greater than 2000, greater than 2500, greater than 3000, or greater than 5000 base pairs (or nucleotides) in length.

As is the case with the sample nucleic acids, the target nucleic acids may be any type of nucleic acid including: double-stranded (ds) nucleic acids, single stranded (ss) nucleic acids, DNA, RNA, cDNA, mRNA, cRNA, tRNA, ribosomal RNA, dsDNA, ssDNA, miRNA, siRNA, circulating nucleic acids, circulating DNA, circulating RNA, cell-free nucleic acids, cell-free DNA, cell-free RNA, circulating cell-free DNA, circulating cell-free RNA, genomic DNA, mitochondrial nucleic acids, or any combination thereof. In some cases, the double-stranded target nucleic acids are natural double-stranded nucleic acids present in the sample prior to any sample processing. In some cases, the double-stranded target nucleic acids are double-stranded as a result of an *in vitro* amplification reaction.

### Nucleic Acid Reagents

Generally, the methods provided herein involve inactivating nucleic acids that have contaminated a nucleic acid assay reagent. The term "nucleic acid assay reagent" is used interchangeably herein with the term "nucleic acid reagent." Non-limiting examples of a nucleic acid reagent include water, solvent (e.g., ethanol, isopropanol), aqueous solution, buffer, elution solution, nucleic acid elution buffer, nucleic acid binding buffer, wash buffer, nucleic acid extraction buffer, hybridization reagent, equilibration buffer, purification reagent, preparation reagent, deparaffinization solution, cell lysis buffer, sample lysis buffer, tissue lysis buffer, viral particle lysis buffer, suspension or resuspension buffer, neutralization buffer, digestion buffer, equilibration buffer, solubilization buffer, DNA lysis buffer, blood/plasma stabilization reagent, blood component separation gel, and PCR reagent. The nucleic acid binding buffer may include nucleic acid binding particles, such as magnetic particles.

A nucleic acid reagent provided herein may contain any component commonly used in molecular or cellular biology lab techniques. For example, the nucleic acid reagent may contain a detergent, surfactant, buffer, preservative, salt, acid, base, sugar, carbohydrate, stabilizer, alcohol or any other component. Specific examples of components within the nucleic acid reagent may include but are not limited to: Tris hydrochloride, EDTA, cetyltrimethyl ammonium bromide, sodium chloride (NaCI), sodium fluoride, sodium phosphate, sodium orhtovanadate, aprotinin, protease inhibitor, dithiothreitol (DTT), HCl, water, ethanol, HEPES, NaOH, Sodium dihydrogen phosphate, KCl, phenol, and (NH₄)₂SO₄, proteinase (e.g., proteinase K), DNase, RNase, phenol, chloroform, guanidine hydrochloride, or sodium iodide. The detergent may be a non-ionic detergent, cationic detergent, anionic detergent, ionic detergent, zwitterionic detergent or other type of detergent. Some particular examples of a detergent within a nucleic acid reagent include but are not limited to: Triton X, Tween, sodium dodecyl sulfate (SDS), Nonidet P-40 (NP-40), sodium deoxycholate, and even dish soap. In some cases, the nucleic acid assay reagent may be phenol-free.

Often, the nucleic acid reagent contains a buffer or other agent to maintain the pH of the reagent. The pH of the nucleic acid reagent may be pH 4, pH 5, pH 6, pH 7, pH 8, pH 9, pH 10, pH 11, pH 12, or higher. The pH of the nucleic acid reagent may fall within a particular pH range such as less than pH 4, pH4-6, pH 5-7, pH 6-9, pH 7-8, pH 9-11, or at least pH 12.

Generally, a nucleic acid reagent provided herein retains all of its original activity or a significant portion of its original activity following an inactivation step provided herein. As such, the nucleic acid reagent may function as well as, or almost as well as, it did prior to the inactivation step. For example, the nucleic acid reagent may retain at least 30%, 40%, 50%, 60%, 70%, 80%, 90%, 95%, or 99% or more of its original functionality or activity. For example, an extraction buffer that is originally able to extract say 90% of the nucleic acids in a sample may retain at least 50% of its activity following an inactivation step provided herein and extract at least 45% of the nucleic acids in a sample following the inactivation step.

In some specific examples, the methods involve the inactivation of contaminants introduced during the course of extracting nucleic acids (e.g., DNA) from cells. Nucleic acid reagents such as cell lysis buffers may be used during this process, particularly lysis buffers containing a detergent or surfactant capable of removing or destroying membrane lipids. The process of DNA extraction from cells may also involve using a buffer containing a protease in order to remove proteins and/or a buffer containing RNase in order to remove RNA.

Nucleic acid (e.g., DNA) extraction from cells may also involve us of purification reagents to remove detergents, proteins, salts and other reagents from the DNA. Such purification reagents may be subjected to any of the contaminant inactivating methods provided herein. In some cases, such purification reagents may comprise ethanol in order to enable ethanol precipitation usually by ice-cold ethanol or isopropanol. In some cases, the ionic strength of such buffers is increased, usually by adding sodium acetate. In some cases, a purification reagent contains phenol and/or chloroform. For phenol-chloroform extraction, phenol denatures proteins in the sample. After centrifugation of the sample, denaturated proteins stay in organic phase while aqueous phase containing nucleic acid is mixed with the chloroform that removes phenol residues from solution. In some cases, DNA isolation is accomplished using phenol buffered to pH 8; in some cases, RNA is isolated using phenol such as an acidic phenol. Purification may also be accomplished via column (or minicolumn) purification. In column purification, the nucleic acid may bind or adsorb to a solid phase (e.g., silica or other), often depending on the pH and the salt content of the buffer. In some cases, a nucleic acid purification reagent may include a chelating agent to sequester divalent cations, such as Mg2+ and Ca2+, which prevents enzymes like DNase from degrading the DNA

In some specific examples, the methods involve the inactivation of contaminants introduced during the course of extracting cell-free nucleic acids (e.g., cfDNA, cfRNA) or circulating cell-free nucleic acids (e.g., circulating cfDNA, circulating cfRNA) from a sample. Generally, such sample is a body fluid such as blood, plasma, serum, urine, cerebrospinal fluid, or the like. In some cases, the sample itself is cell-free (e.g., plasma, serum).

Extraction of cell-free nucleic acids from cell-free body fluids (e.g., plasma, serum, urine, cerebrospinal fluid) may be accomplished by a number of approaches include by using a commercial kit such as the QIAamp circulating nucleic acid kit sold by Qiagen. Often, the procedure involves the use of mini columns (e.g., QIAamp mini-columns) with a vacuum manifold. The procedure may be multiplexed over multiple samples, e.g., at least 1 sample, at least 5 samples, at least 10 samples, etc.

Extraction of cell-free circulating nucleic acids may involve one or more of the following steps: lysis, binding to a column or to nucleic acid binding particles in nucleic acid binding buffer, washing the column, and elution of nucleic acids from the column. The nucleic acid binding particles may include magnetic particles. The methods provide herein include methods of inactivating reagents used during one or more of these steps. A lysis reagent may be used to release cell-free circulating nucleic acids from proteins or lipids bound to the nucleic acids or from vesicles enveloping the cell-free nucleic acids. The lysis may be under highly denaturing conditions. In some cases, the lysis performed under high heat conditions. The lysis reagent may include protease (e.g., proteinase k) to degrade proteins as well as agents to de-activate DNase and RNase, particularly endogenous DNase and RNase. In some cases, the buffer is Buffer ACL (Qiagen) and/or Buffer ATL (Qiagen). Buffer ATL or other special buffer may be used during the process of extracting nucleic acids form urine samples or to extract miRNA. In some cases carrier RNA is added to the lysis buffer, e.g., Buffer ACL.

The binding step may include subjecting the sample to a binding buffer reagent (e.g., Buffer ACB) to promote binding of the nucleic acids to a column, e.g., silica membrane. The binding buffer reagent may be prepared with optimized salt and pH conditions to ensure that proteins and other contaminants do not stick to the column. Often, the binding buffer includes an alcohol such as isopropanol. Vacuum or other pressure may be used to draw the sample through the column. Then, a wash buffer reagent (e.g., buffer ACW) may be used to wash the column for one or more wash steps, e.g., 1, 2, 3, or 4 or more wash steps. The wash buffer may comprise ethanol. The nucleic acids may then be eluted from the column using an elution reagent such as Buffer AVE, which may be equilibrated to a certain temperature, e.g., room temperature. The protocol may vary depending on the nature of a sample. For example, a protocol for removing cell-free nucleic acids from urine may be different from that to remove cell-free nucleic acids from plasma.

### Contaminants

Contaminants within a nucleic acid reagent include any contaminant introduced into the reagent by exposure to the environment including during manufacture of the reagent or collection equipment. The contaminant may be nucleic acids or may be a cell such as a bacterial cell, or any other natural or synthetic material containing nucleic acids. In some instances, the contaminant is derived from a human, such as a lab worker or plant worker. In some instances, the contaminant is derived from a non-human organism, particularly a pathogen such as a microbe, bacterium, virus, fungus, parasite, worm, or any combination thereof. In some cases, the target nucleic acids and the contaminant nucleic acids may be derived from the same microbial species. For example, the target nucleic acids and the contaminant nucleic acids both are bacterial nucleic acids. In some cases, the target nucleic acids and the contaminant nucleic are from identical bacteria, e.g., both the target nucleic acids and the contaminant nucleic acids are *Escherichia coli* bacteria. In another example, the target nucleic acids and the contaminant nucleic acids are both fungal nucleic acids. In some cases, the target nucleic acids and the contaminant nucleic are from identical types of fungi.

In some cases, contaminant nucleic acids may include nucleic acids derived from one or more eukaryotic species, one or more prokaryotic species, one or more viral species, one or more fungal species, or any combination thereof. In some cases, the contaminant nucleic acids are nucleic acids from soil bacteria. In some cases, contaminant nucleic acids may include nucleic acids derived from *Propionibacterium acnes,* Bradyrhizobium sp. S23321, *Bradyrhizobium diazoefficiens, Bradyrhizobium japonicum,* Acidovorax sp. KKS102, Bradyrhizobium sp. BTAi1, *Acidovorax temperans, Delftia sp. Csl-4, Delftia acidovorans, Methylobacterium acidovorans, Rhodopseudomonas palustris, Serratia marcescens, Acinetobacter junii, Acinetobacter johnsonii, Chryseobacterium indologenes* or any combination thereof. In some cases, contaminant nucleic acids may include nucleic acids derived from genera *Bradyrhizobium, Rhizobium*/*Agrobacterium, Sphingomonas, Burkholderia, Ralstonia, Pseudomonas, Stenotrophomonas, Flavobacterium, Herbaspirillum, Methylobacteria, Delftia, Rhodopseudomonas, Serratia, Acinetobacter, Chryseobacerium* or any combination thereof. In some cases, contaminant nucleic acids may include nucleic acids derived from *Bradyrhizobium* or *Bradyrhizobium sp. DFCI-1.* In some cases, contaminant nucleic acids may include nucleic acids derived from *Escherichia coli.* In some cases, contaminant nucleic acids may include nucleic acids derived from *Bos* (e.g., cow), *Sus* (e.g., pig), *Gallus* (e.g., chicken), or any combination thereof.

As is the case with the sample nucleic acids, the contaminant nucleic acids may be any type of nucleic acid including: double-stranded (ds) nucleic acids, single stranded (ss) nucleic acids, DNA, RNA, cDNA, mRNA, cRNA, tRNA, ribosomal RNA, dsDNA, ssDNA, miRNA, siRNA, circulating nucleic acids, circulating DNA, circulating RNA, cell-free nucleic acids, cell-free DNA, cell-free RNA, circulating cell-free DNA, circulating cell-free RNA, genomic DNA, mitochondrial nucleic acids, or any combination thereof. In some examples, the contaminant nucleic acids can be in the form of double stranded DNA fragments at low concentrations in various solutions.

The length of contaminant nucleic acids can vary. In some cases, contaminant nucleic acids may be about or at least about 20, 30, 40, 50, 60, 70, 80, 90, 100, 110, 120, 130, 140, 150, 160, 170, 180, 190, 200, 250, 300, 350, 400, 450, 500, 750, 1000, 1500 or 2000 nucleotides in length. In some cases, contaminant nucleic acids may be up to about 20, 30, 40, 50, 60, 70, 80, 90, 100, 110, 120, 130, 140, 150, 160, 170, 180, 190, 200, 250, 300, 350, 400, 450, 500, 750, 1000, 1500 or 2000 nucleotides in length. In some cases, the contaminant nucleic acids may be at least about 100 nucleotides in length. In some cases, the contaminant nucleic acids may be at least about 75 nucleotides in length. In some cases, the contaminant nucleic acids may be at least about 50 nucleotides in length. In some cases, the contaminant nucleic acids may be at least about 40 nucleotides in length.

In some cases, the contaminant nucleic acids may make up about or at least about 0.00001%, 0.00005%, 0.0001%, 0.0005%, 0.001%, 0.005%, 0.01%, 0.05%, 0.1%, 0.5%, or 1% of total sequencing reads or read pairs. In some cases, the concentration of contaminant nucleic acids may be no more than 0.00000001%, 0.00000005%, 0.0000001%, 0.0000005%, 0.000001%, 0.000005%, 0.00001%, 0.00005%, 0.0001%, 0.0005%, 0.001%, 0.005%, 0.01%, 0.05%, 0.1%, 0.5%, or 1% of total sequencing reads or read pairs. In some cases, the concentration of contaminant nucleic acids may be within the range between about 0.000001% and 1%, between about 0.000001% and about 0.1%, between about 0.000005% and about 0.1%, or between about 0.000005% and 0.05% of total sequencing reads or read pairs.

In some cases, the concentration of contaminant nucleic acids in the nucleic acid reagent may be about or at least about 1 pM, 10 pM, 100 pM, 1 nM, 10 nM, 100 nM, 1 µM, 10 µM, or 100 µM. In some cases, the concentration of contaminant nucleic acids may be less than or equal to about 1 pM, 10 pM, 100 pM, 1 nM, 10 nM, 100 nM, 1 µM, 10 µM, or 100 µM.

In some cases, the degree of contaminant nucleic acid reduction or inactivation is about or at least about 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.5%, 99.9%, 99.95%, or 99.99%. In some cases, the degree of contaminant nucleic acid reduction or inactivation is up to about 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.5%, 99.9%, 99.95%, or 99.99%. In some cases, the degree of contaminant nucleic acid reduction or inactivation is within a range of, e.g., 50% to 80%, 60% to 90%, 60% to 100%, or 70%-90%.

### Inactivation of contaminant nucleic acids

The contaminants within the nucleic acid reagents may be inactivated or removed by any of a number of methods or approaches. In some cases, the contaminants are inactivated by exposure to radiation (e.g., UV, gamma irradiation); in some cases, the contaminants are inactivated by exposure to heat; in some cases, the contaminants are inactivated by exposure to a chemical; and in some cases, enzymes such as nucleases are used to degrade the contaminant nucleic acids. Examples of such types of removal include but are not limited to: replacing contaminated reagents with decontaminated or less contaminated reagents, washing solid materials with clean solutions such as water, filtering nucleic acid reagents, or otherwise treating solutions with nucleic acid binding materials. An additional method is to bioinformatically subtract reads determined to be common contaminants (for example, from parallel sterile water or buffer input control samples).

The methods provided in this disclosure include methods of inactivating contaminants in specific nucleic acid reagents, such as extraction buffers, lysis buffers, and purification buffers. For example, the method may involve applying heat, non-ionizing radiation, or gamma radiation to an extraction buffer, lysis buffer and/or purification buffer in order to inactivate contaminants present in an extraction buffer, lysis buffer, purification buffer, PCR buffer or other buffer used during the course of an assay.

In some cases, it may not be necessary to inactivate contaminants used at each step of a particular assay such as a sequencing assay. For example, inactivation of contaminating nucleic acids in PCR buffers may not be necessary in cases where the target DNA or other nucleic acid is already attached to adapters prior to introduction of the PCR buffer. As such, it may not be necessary to inactivate contaminating nucleic acids in a buffer introduced to an assay after an adapter attachment step. In some cases, it may not be necessary to inactivate contaminating nucleic acids in particular buffers, such as ligase buffers, since ligase buffers are generally not a major source of contaminating nucleic acids.

### Heat inactivation of contaminant nucleic acids

Application of heat to a reagent or nucleic acid analysis buffer may be used in the methods provided herein to inactivate signal from contaminant nucleic acids. For example, heating a reagent or nucleic acid analysis buffer may melt or denature some or all double stranded DNA (dsDNA) contaminant fragments present in the reagent or buffer and convert them into single stranded DNA (ssDNA). After heating, solutions may be cooled (often, rapidly cooled) to a lower temperature such as their storage temperature, making it unlikely for a single strand to be able to find its complementary strand at the low concentrations at which contaminant nucleic acids are present. The single stranded DNA or mispaired dsDNA fragments may not be efficiently converted into molecules that can be ligated or attached to the adapters or amplified or sequenced, thereby reducing their concentration in the final library.

The heating treatment may be effective in inactivating at least a portion of contaminant nucleic acids. The effectiveness of the heating treatment can depend on the nature of the contaminants and/or on the composition of the reagents or buffers. For example, the heating treatment may not be as effective on ssDNA when compared with dsDNA contaminant nucleic acids. The effectiveness of the heating treatment can be improved by evenly distributing the heat during the heating treatment. For example, the heating treatment can be more effective when the reagents are agitated or rotated during the heat treatment.

The heating treatment can be effectively used for applications such as sequencing. The method can include subjecting a nucleic acid analysis buffer to the heating treatment to inactivate at least a portion of the contaminant nucleic acids to produce a decontaminated nucleic acid analysis buffer. The heating treatment denatures dsDNA into ssDNA. The decontaminated nucleic acid analysis buffer can then be used with a sample of target nucleic acids. The target nucleic acids in the sample can then be attached with double stranded adapters to produced tagged target nucleic acids. The double stranded adapted attaches to the double stranded target nucleic acids, not to denatured ssDNA. The tagged target nucleic acids are then subjected to a sequencing assay. In some cases, the method may also include performing an amplification reaction after attaching the double stranded adapters to the target nucleic acids.

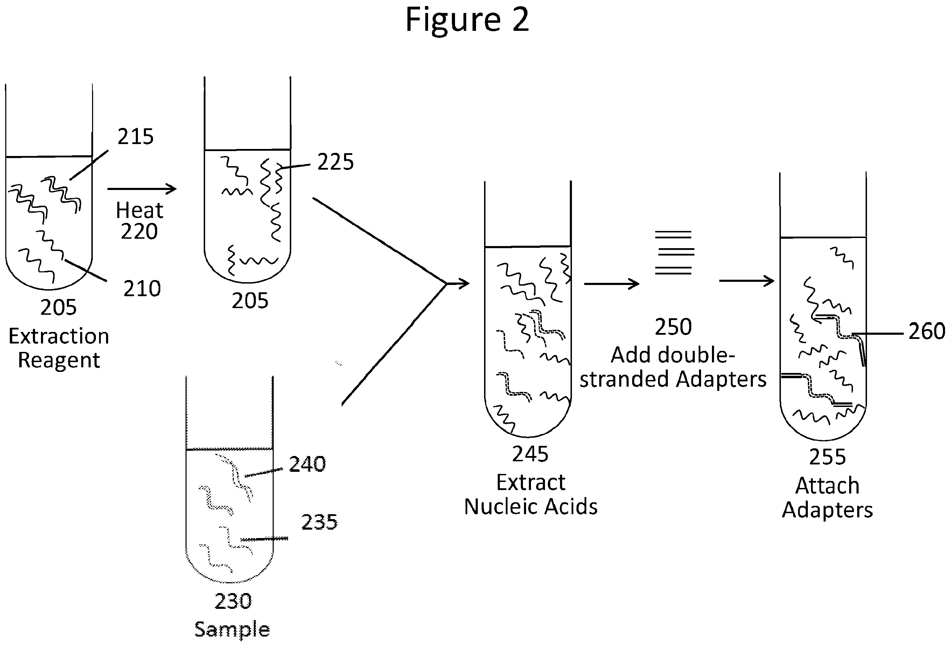

**Fig. 2** provides a basic schematic depicting methods provided herein, particularly methods for removing contaminant nucleic acids from a nucleic acid buffer using heat. In some cases, a method provided herein may include removing or reducing contaminant nucleic acids present in a nucleic acid analysis buffer, such as an extraction buffer **205.** In some cases, the extraction buffer is specially formulated to extract cell-free circulating nucleic acids from a sample. The contaminant nucleic acid can be a single-stranded nucleic acid **210** and/or double-stranded nucleic acid **215.** In some cases, the contaminant nucleic acids are from different sources of contaminants, such as different bacteria. The extraction buffer can be subjected to a heating treatment **220.** The heating treatment may denature double-stranded nucleic acids **215** into single-stranded nucleic acids **225.** As a result, all or most of the contaminant nucleic acids in the nucleic acid buffer (e.g., extraction buffer) may be present in a single-stranded form. In some cases, the extraction buffer **205** can be cooled to a room temperature prior to contacting a biological sample **230** with the extraction buffer **205.** In other cases, the extraction buffer is not significantly cooled prior to contacting the biological sample. The biological sample may include a single-stranded nucleic acid **235** and/or a double-stranded nucleic acid **240.** As shown in **245,** the nucleic acids in the biological sample **230** can be extracted using the extraction buffer **205.** Double-stranded adapters **250** can be added to the extracted nucleic acids **245.** In other cases, the double-stranded adapters **250** can be Y-shaped (not shown). In some cases, the double-stranded adapters **250** may be designed such that they are capable of being attached to double-stranded nucleic acids, but not single-stranded nucleic acids. As shown in **255,** the adapters may be attached (e.g., ligated) at the ends of the double stranded sample nucleic acids **260.** In some cases, particularly where the adapters are designed to attach to double-stranded nucleic acids, the adapters are not attached to the single-stranded contaminants derived from the extraction reagent. The resulting tagged nucleic acids may be sequenced in a sequencing assay, e.g. Next Generation Sequencing assay. In some cases, the tagged nucleic acids are amplified, e.g., with a primer that recognizes a primer binding site present in the adapter. In some cases, the adapters are sequencing adapters that are recognized by sequencing device. The sequencing adapters may be attached (e.g., via ligation or primer extension) directly to the double-stranded nucleic acids originating from the sample. In some cases, the sequencing adapters are attached to the amplification adapters prior to or following an amplification step. The heating treatment followed by attaching double-stranded adapters can result in elimination or reduction of contaminant nucleic acids in sequencing libraries, ultimately resulting in improved and more-accurate identification of target nucleic acids that originated in the sample.

In some cases, heating can occur at a temperature of about or at least about 30, 35, 40, 45, 50, 55, 60, 65, 70, 75, 80, 85, 90, 91, 92, 93, 94, 95, 96, 97, 98, 99, 100, 110, 120, 130, or 140 °C. In some cases, heating can occur at a temperature of up to about 30, 35, 40, 45, 50, 55, 60, 65, 70, 75, 80, 85, 90, 91, 92, 93, 94, 95, 96, 97, 98, 99, 100, 110, 120, 130, or 140 °C. In some examples, heating can occur at a temperature of at least about 80°C. In some examples, heating can occur at a temperature of at least about 90°C. In some examples, heating can occur at a temperature of at least about 95°C.

In some cases, heating can occur for a duration of about or at least about 1, 5, 10, 15, 20, 30, 35, 40, 50, or 55 seconds; about or at least about 1, 5, 10, 15, 20, 30, 40, or 50 minutes; about or at least about 1, 2, 3, 4, 5, 6, 7, 8, 9, 10, 11, 12, 13, 14, 15, 16, 17, 18, 19, 20, 21, 22, or 23 hours; or about or at least about 1, 2, 3, 4, 5, 6, 7, 8, 9, or 10 days. In some cases, heating can occur for a duration of up to about 1, 5, 10, 15, 20, 30, 40, or 50 seconds; up to about 1, 5, 10, 15, 20, 30, 40, or 50 minutes; up to about 1, 2, 3, 4, 5, 6, 7, 8, 9, 10, 11, 12, 13, 14, 15, 16, 17, 18, 19, 20, 21, 22, or 23 hours; or up to about 1, 2, 3, 4, 5, 6, 7, 8, 9, or 10 days. In some particular examples, a buffer or reagent is subjected to a heat treatment for at least 30 minutes in order to remove all or a substantial portion of contaminating nucleic acids that may be present in the buffer or reagent.

In some cases, heating can occur in a heated bath, water bath, oil bath, silicone bath, sand bath, circulating bath, non-circulating bath, incubator, hot plate, heating element, oven, flame, Bunsen burner, and any combination thereof. Heating may occur with mixing, stirring, shaking, rotation, sonication, and/or homogenizing.

In some cases, heated solutions (e.g., nucleic acid analysis buffer) or reagents are cooled to a temperature of about or at least about -80, -50, -40, -30, -20, -15, -10, -5, 0, 1, 2, 3, 4, 5, 10, 15, 20, 25, 30, 35, 36, 37, 38, 39, or 40 °C or room temperature. In some cases, heated solutions or reagents are cooled to a temperature of up to about -80, -50, -40, -30, -20, -15, -10, -5, 0, 1, 2, 3, 4, 5, 10, 15, 20, 25, 30, 35, 36, 37, 38, 39, or 40 °C or room temperature. In some cases, heated solutions (e.g., nucleic acid analysis buffer) or reagents are cooled to a temperature less than 90 °C, less than 85 °C, less than 80 °C, less than 75 °C, less than 70 °C, less than 65 °C, or less than 60 °C. In some cases, the heated solutions (e.g., nucleic acid analysis buffer) or reagents are cooled to a temperature between about 50-65 °C. In some cases, the heated solutions (e.g., nucleic acid analysis buffer) or reagents are cooled to a temperature between about 75-80 °C. In some cases, the heated solutions (e.g., nucleic acid analysis buffer) or reagents are cooled to a temperature between about 20-25 °C.

In some cases, cooling can occur for a duration (e.g., amount of time to reach the cooling temperature from the heating temperature) of about or at least about 1, 5, 10, 15, 20, 30, 40, or 50 seconds; 1, 5, 10, 15, 20, 30, 40, or 50 minutes; about or at least about 1, 2, 3, 4, 5, 6, 7, 8, 9, 10, 11, 12, 13, 14, 15, 16, 17, 18, 19, 20, 21, 22, or 23 hours; or about or at least about 1, 2, 3, 4, 5, 6, 7, 8, 9, or 10 days. In some cases, cooling can occur for a duration of up to about 1, 5, 10, 15, 20, 30, 40, or 50 seconds; up to about 1, 5, 10, 15, 20, 30, 40, or 50 minutes; up to about 1, 2, 3, 4, 5, 6, 7, 8, 9, 10, 11, 12, 13, 14, 15, 16, 17, 18, 19, 20, 21, 22, or 23 hours; or up to about 1, 2, 3, 4, 5, 6, 7, 8, 9, or 10 days.

In some cases, the cooling can be rapid cooling in which the reagent or nucleic acid analysis buffer is cooled to a certain temperature (e.g., room temperature) within a short period of time, e.g., less than 15, 10, 5, 4, or 2 seconds. In some cases, the certain temperature is a temperature less than the denaturing temperature, or significantly less than the denaturing temperature. For example, the certain temperature may be less than 90 °C, less than 85 °C, less than 80 °C, less than 75 °C, less than 70 °C, less than 65 °C, or less than 60 °C.

The reagent or nucleic acid analysis buffer may be used after a heating step or a cooling step. In some cases, the reagent or nucleic acid analysis buffer is used immediately after the heating or cooling step (e.g., less than 10 minutes, less than 5 minutes, or less than 1 minute following the heating or cooling step). In comes cases, the reagent or nucleic acid analysis buffer is used after a certain time period has elapsed after the cooling or heating step (e.g., greater than 10 minutes, 30 minutes, 1 hour, 5 hours, 12 hours, 1 day, 10 days, 1 month, 2 months, 3 months or longer following the heating or cooling step).

In some cases, cooling can occur in an ice bath, refrigerator, freezer, anti-griddle, flash freeze, semi-freeze, dry ice, water bath, oil bath, silicone bath, sand bath, circulating bath, non-circulating bath, incubator, and any combination thereof. Cooling may occur with mixing, stirring, shaking, rotation, sonication, and/or homogenizing.

In some cases, one or more additives may be added prior to or during heating to modulate nucleic acid melting temperature, boiling point, and/or pH. For example, an additive may be a salt such as a magnesium or sodium containing salt (e.g., MgCl₂, NaCl) or a buffering agent.

In some cases provided herein, decontaminated buffer or reagent may be used to clean surfaces or labware (e.g., benchtop, counter, fume hood, table, glassware, plastic, tube, Falcon tube, Eppendorf tube, pipette tip, pipette, syringe, spatula, needle, plate, well, instrument, glove, weighing boat, weighing paper, column, container). In some cases, surfaces or labware can be rinsed with a buffer or water that has been heated, or filled with a buffer or water, heated, and dried.

In some cases, an approach other than heating may be used to inactivate the contaminating nucleic acids in a reagent or nucleic acid analysis buffer. Such approach may be performed alone or in combination with heating. Examples of such approach may include use of a chemical denaturant such as an acid, base, organic solvent, cross-linking reagent, chaotropic agent, and/or di-sulfide bond-reducing agent.

### Irradiation to inactivate contaminant nucleic acids

Contaminating nucleic acid fragments in reagents (e.g., nucleic acid sequencing preparation materials) or from other elements of the environment may be inactivated with irradiation, for example using ultraviolet light (UV) or gamma irradiation. DNA damaged with UV may produce bulky DNA lesions, such as pyrimidine dimers. The DNA lesions may block the DNA polymerases used in library preparation and in the sequencing reaction and thus DNA damaged with UV may not be detected. Likewise, gamma irradiation can damage the bases of the DNA and prevent copying and/or detection, or it can break the DNA backbone and with a high enough dose reduce the DNA fragment sizes below the detection limit of the extraction or library preparation methods. Reagents and other materials used in preparing a sample for sequencing "cleaned" in this way may still contain contaminating nucleic acids, but these inactivated or damaged nucleic acids may avoid detection by the sequencing system.

Non-limiting examples of electromagnetic radiation include gamma rays, X-rays (e.g., hard X-rays, soft X-rays), and ultraviolet (e.g., ultraviolet A, ultraviolet B, ultraviolet C, extreme ultraviolet, vacuum ultraviolet, far ultraviolet, middle ultraviolet, near ultraviolet). In some cases, the wavelength of the electromagnetic radiation is about or at least about 1 pm, 5 pm, 10 pm, 50 pm, 100 pm, 500 pm, 1 nm, 5 nm, 10 nm, 50 nm, 100 nm, 500 nm, or 1 µm. In some cases, the wavelength of the electromagnetic radiation is 254 nm. In some cases, the electromagnetic radiation is ultraviolet C.

In some cases, the dose of electromagnetic radiation is about or at least about 1,000; 2,000; 3,000; 4,000; 5,000; 6,000; 7,000; 8,000; 9,000; 10,000; 11,000; 12,000; 13,000; 14,000; or 15,000 J/m². In some cases, the dose of electromagnetic radiation is up to about 1,000; 2,000; 3,000; 4,000; 5,000; 6,000; 7,000; 8,000; 9,000; 10,000; 11,000; 12,000; 13,000; 14,000; or 15,000 J/m². In some cases, the dose of electromagnetic radiation is about or at least about 0.1, 0.2, 0.3, 0.4, 0.5, 0.6, 0.7, 0.8, 0.9, 1, 1.5, 2, 2.5, 3, 3.5, 4, 4.5, 5, 6, 7, 8, 9, or 10 kGy. In some cases, the dose of electromagnetic radiation is up to about 0.1, 0.2, 0.3, 0.4, 0.5, 0.6, 0.7, 0.8, 0.9, 1, 1.5, 2, 2.5, 3, 3.5, 4, 4.5, 5, 6, 7, 8, 9, or 10 kGy.

In some cases, contaminant nucleic acids can be irradiated at about or at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 20, 25, 30, 35, 40, 45, or 50 cm from a source of electromagnetic radiation. In some cases, contaminant nucleic acids can be irradiated at up to about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 20, 25, 30, 35, 40, 45, or 50 cm from a source of electromagnetic radiation.

In some cases, irradiation can occur for a duration of about or at least about 1, 5, 10, 15, 20, 30, 40, or 50 seconds; 1, 5, 10, 15, 20, 30, 40, or 50 minutes; 1, 2, 3, 4, 5, 6, 7, 8, 9, 10, 11, 12, 13, 14, 15, 16, 17, 18, 19, 20, 21, 22, or 23 hours; or 1, 2, 3, 4, 5, 6, 7, 8, 9, or 10 days. In some cases, irradiation can occur for a duration of up to about 1, 5, 10, 15, 20, 30, 40, or 50 seconds; 1, 5, 10, 15, 20, 30, 40, or 50 minutes; 1, 2, 3, 4, 5, 6, 7, 8, 9, 10, 11, 12, 13, 14, 15, 16, 17, 18, 19, 20, 21, 22, or 23 hours; or 1, 2, 3, 4, 5, 6, 7, 8, 9, or 10 days.

The effectiveness of the irradiation can depend on various factors. For example, the effectiveness of the gamma irradiation can depend on the composition of the buffers or reagents. The gamma irradiation may not be effective in inactivating contaminant nucleic acids from reagents or buffers with radiation quenchers, such as salts. Thus, the gamma irradiation may be more effective on low-salt buffers or on water. The irradiation treatment may be effective in inactivating both dsDNA and ssDNA contaminating nucleic acids. The irradiation treatment may be more effective than the heating treatment when inactivating larger DNA fragments such as DNA fragments greater than 75 base pairs in length. Irradiation treatment may be particularly useful to inactivating single-stranded contaminating nucleic acids.

### Enzymatic inactivating agents

Contaminant nucleic acids can be inactivated by treatment with one or more enzymes, such as nucleases. A nuclease is generally an enzyme capable of cleaving the phosphodiester bonds between the nucleotide subunits of nucleic acids. Endonucleases and exonucleases are types of nucleases. Endonucleases include, but are not limited to deoxyribonuclease (e.g., DNase I), heat-labile dsDNase, restriction enzymes, and Cas proteins (e.g., Cas9). In some cases, after treatment of the contaminant nucleic acids, an endonuclease or other enzyme can be removed or inactivated (e.g., by heating).

Enzymatic inactivating agents can be effective in inactivating both ssDNA and dsDNA contaminating nucleic acids. The effectiveness of the enzymatic agents can depend on various factors. For example, the enzymatic treatment may not be effective if the reagents or buffers contain ingredient(s) that denature protein. The enzymatic treatment may need a large quantity of enzyme for effectively inactivating contaminating nucleic acids.

In some cases, a method of this disclosure does not involve contaminating nucleic acids using enzymatic treatment. For example, the method may not involve contacting a reagent with an enzyme such as endonuclease, DNAse I, and/or heat-labile DNase enzyme in order to inactivate contaminating nucleic acids present in the reagent.

### Physical removal of contaminant nucleic acids

In some cases, contaminant nucleic acids may be physically removed from a nucleic acid reagent. In some cases, a nucleic acid reagent may be tested for contaminants and replaced with a new reagent if contaminants are found. Physical removal may also be accomplished by running the reagent through a filter to remove nucleic acid contaminants. In some cases, a binding agent, e.g., a protein, may be added to the reagent and then the nucleic acid-protein complex is removed, e.g., via filtration. Washing solid materials with clean solutions such as water may also result in physical removal of contaminants from a solid surface.

### Bioinformatic removal of contaminant nucleic acids

The methods provided herein include methods of bioinformatically removing contaminant nucleic acids when sequencing data is analyzed. The methods provided herein enable one to distinguish between DNA that arose from an infection or normal commensal organisms and DNA from environmental contamination (EC). In practice, the EC alone may give rise to DNA that maps to hundreds of different species, with a very broad range of abundances.

In order to study the environmental contribution to the non-host (e.g., non-human) DNA signal, negative controls (NCs) can be sequenced, which can start as water samples rather than plasma. Larger collections of these NCs may be occasionally sequenced, comprising "global baselines". For example, a larger collection of NCs may comprise about or at least about 5, 10, 15, 20, 25, 30, 40, 50, 60, 70, 80, 90, 100, 200, 300, 400, 500, or 1000 NCs. In some cases, a larger collection of NCs may comprise up to about 5, 10, 15, 20, 25, 30, 40, 50, 60, 70, 80, 90, 100, 200, 300, 400, 500, or 1000 NCs. Then, on each sequencing run, alongside the clinical samples of interest, "local baselines", or smaller collections of NCs, can be sequenced. For example, a smaller collection of NCs may comprise about or at least about 1, 2, 3, 4, 5, 6, 7, 8, 9, 10, 15, 20, 25, 30, 40, or 50 NCs. In some cases, a smaller collection of NCs may comprise up to about 1, 2, 3, 4, 5, 6, 7, 8, 9, 10, 15, 20, 25, 30, 40, or 50 NCs, such as 1-5 NCs or 2-3 NCs. The global baseline can be taken with a protocol that has a manageably low level of EC. Then, each local baseline may be compared with the global baseline to make sure that EC levels have not gone up significantly. Then, for each clinical sample, the inferred abundance of each taxon may be compared to that of the local baseline. In order to account for the natural variations in library size, the level of a spike-in molecule can be used to scale abundances in the local baseline to a level appropriate in the clinical sample. A simple counting statistics model can be used to compare the abundance of each taxon in the clinical sample to the local baseline, and only taxa with abundances that deviate very strongly from the EC expectation can be reported.

### Combination of treatments

The contaminating nucleic acids can be removed using either a single treatment or a combination of two or more treatments. For example, heating treatment may be combined with irradiation treatment, such as UV, for effectively inactivating contaminating nucleic acids. The combined use of heating and non-ionizing irradiation treatments of a reagent (e.g., extraction buffer, purification buffer, lysis buffer, etc.) may effectively inactivate most of the contaminating nucleic acids. For example, short contaminating nucleic acids can be inactivated by applying heat to the nucleic acid analysis buffer and long nucleic acids can be inactivated by applying non-ionizing radiation (e.g. UV) to the nucleic acid analysis buffer.

### Sequencing

This disclosure provides methods of analyzing nucleic acids produced using a fully or partially decontaminated reagent described herein such as an extraction reagent. Such analytical methods can include sequencing the nucleic acids as well as bioinformatic analysis of the sequencing results. Nucleic acids obtained using the methods described herein generally have reduced amounts contaminant nucleic acids and therefore produce superior sequencing results when analyzed by a sequencing assay or subjected to other downstream analyses. The nucleic acids produced according the present methods may be analyzed to obtain various types of information including genomic, epigenetic (e.g., methylation), and RNA expression. Methylation analysis can be performed by, for example, conversion of methylated bases followed by DNA sequencing. RNA expression analysis can be performed by, for example, polynucleotide array hybridization.

In preferred embodiments, the sequencing is performed using a next generation sequencing assay. As used herein, the term "next generation" generally refers to any high-throughput sequencing approach including, but not limited to one or more of the following: massively-parallel signature sequencing, pyrosequencing (e.g., using a Roche 454 sequencing device), Illumina (Solexa) sequencing, sequencing by synthesis (Illumina), Ion torrent sequencing, sequencing by ligation (e.g., SOLiD sequencing), single molecule real-time (SMRT) sequencing (e.g., Pacific Bioscience), polony sequencing, DNA nanoball sequencing, and heliscope single molecule sequencing (Helicos Biosciences). In some cases, the sequencing assay uses nanopore sequencing. In some cases, the sequencing assay includes some form of Sanger sequencing. In some cases, the sequencing involves shotgun sequencing; in some cases, the sequencing includes bridge PCR.

In some cases, the sequencing assay comprises a Gilbert's sequencing method. In such approach, nucleic acids (e.g., DNA) are chemically modified and then cleaved at specific bases. In some cases, a sequencing assay comprises dideoxynucleotide chain termination or Sanger-sequencing.

A sequencing-by-synthesis approach may be used in the methods provided herein. In some cases, fluorescently-labeled reversible-terminator nucleotides are introduced to clonally-amplified DNA templates immobilized on the surface of a glass flowcell. During each sequencing cycle, a single labeled deoxynucleoside triphosphate (dNTP) may be added to the nucleic acid chain. The labeled terminator nucleotide may be imaged when added in order to identify the base and may then be enzymatically cleaved to allow incorporation of the next nucleotide. Since all four reversible terminator-bound dNTPs (A, C, T, G) are generally present as single, separate molecules, natural competition may minimize incorporation bias.

In some cases, a method called Single-molecule real-time (SMRT) is used. In such approach, nucleic acids (e.g., DNA) are synthesized in zero-mode wave-guides (ZMWs), which are small well-like containers with capturing tools located at the bottom of the well. The sequencing is performed with use of unmodified polymerase (attached to the ZMW bottom) and fluorescently labelled nucleotides flowing freely in the solution. The fluorescent label is detached from the nucleotide upon its incorporation into the DNA strand, leaving an unmodified DNA strand. A detector such as a camera may then be used to detect the light emissions; and the data may be analyzed bioinformatically to obtain sequence information.

In some cases, sequencing by ligation approach is used to sequence the nucleic acids in a sample. One example is the next generation sequencing method of SOLiD (Sequencing by Oligonucleotide Ligation and Detection) sequencing (Life Technologies). This next generation technology may generate hundreds of millions to billions of small sequence reads at one time. The sequencing method may comprise preparing a library of DNA fragments from the sample to be sequenced. In some cases, the library is used to prepare clonal bead populations in which only one species of fragment is present on the surface of each bead (e.g., magnetic bead). The fragments attached to the magnetic beads may have a universal PI adapter sequence attached so that the starting sequence of every fragment is both known and identical. In some cases, the method may further involve PCR or emulsion PCR. For example, the emulsion PCR may involve the use of microreactors containing reagents for PCR. The resulting PCR products attached to the beads may then be covalently bound to a glass slide. A sequencing assay such as a SOLiD sequencing assay or other sequencing by ligation assay may include a step involving the use of primers. Primers may hybridize to the P1 adapter sequence or other sequence within the library template. The method may further involve introducing four fluorescently labelled di-base probes that compete for ligation to the sequencing primer. Specificity of the di-base probe may be achieved by interrogating every first and second base in each ligation reaction. Multiple cycles of ligation, detection and cleavage may be performed with the number of cycles determining the eventual read length. In some cases, following a series of ligation cycles, the extension product is removed and the template is reset with a primer complementary to the n-1 position for a second round of ligation cycles. Multiple rounds (e.g., 5 rounds) of primer reset may be completed for each sequence tag. Through the primer reset process, each base may be interrogated in two independent ligation reactions by two different primers. For example, the base at read position 5 is assayed by primer number 2 in ligation cycle 2 and by primer number 3 in ligation cycle 1.

The methods provided herein may include use of a system such as a system that contains a nucleic acid sequencer (e.g., DNA sequencer, RNA sequencer) for generating DNA or RNA sequence information. The system may include a computer comprising software that performs bioinformatic analysis on the DNA or RNA sequence information. Bioinformatic analysis can include, without limitation, assembling sequence data, detecting and quantifying genetic variants in a sample, including germline variants and somatic cell variants (e.g., a genetic variation associated with cancer or pre-cancerous condition, a genetic variation associated with infection).

Sequencing data may be used to determine genetic sequence information, ploidy states, the identity of one or more genetic variants, as well as a quantitative measure of the variants, including relative and absolute relative measures.

In some cases, sequencing of the genome involves whole genome sequencing or partial genome sequencing. The sequencing may be unbiased and may involve sequencing all or substantially all (e.g., greater than 70%, 80%, 90%) of the nucleic acids in a sample. Sequencing of the genome can be selective, e.g., directed to portions of the genome of interest. For example, many genes (and mutant forms of these genes) are known to be associated with various cancers. Sequencing of select genes, or portions of genes may suffice for the analysis desired. Polynucleotides mapping to specific loci in the genome that are the subject of interest can be isolated for sequencing by, for example, sequence capture or site-specific amplification.

### Applications

The methods provided herein may be used to detect infections or diseases in patient samples, such as human blood samples. The methods may be used to detect rare microbial nucleic acid fragments in samples that are predominantly made up of human nucleic acids. For example, cell-free DNA (cfDNA) in blood consists mostly of DNA fragments derived from the host but also contains a small amount of fragments from microbes in the body. Extraction of the cfDNA followed by deep sequencing (e.g., next-generation sequencing or NGS) generates millions or billions of sequence reads that can be mapped against host and non-host genome databases. For samples in which the non-host reads are a very small proportion of the total, the methods provided herein can improve the sensitivity and specificity of the assay, which would otherwise be compromised by contaminating nucleic acid fragments introduced by exposure to the environment, especially from contaminated reagents.

The methods provided herein may be used to detect, monitor, diagnose, prognose, treat, or prevent a large variety of diseases and disorders. In particular, the methods may be used to detect one or more target nucleic acid derived from a pathogen associated with an infectious disease or disorder. Exemplary diseases and disorders include any disease or disorder associated with an infection, e.g., sepsis, pneumonia, tuberculosis, HIV infection, hepatitis infection (e.g., Hep A, B, or C), human papilloma virus (HPV) infection, chlamydial infection, syphilitic infection, Ebola infection, staphylococcus aureus infection, or influenza. The methods provided herein are particularly useful for detecting infections by drug-resistant microbes, including multidrug resistant microbes, or microbes that are not readily cultured or typically tested for. Some non-limiting examples of diseases and disorders that may be detected with the present methods include: cancer, dilated cardiomyopathy, Guillain-Barre syndrome, multiple sclerosis, tuberculosis, anthrax poisoning, sleeping sickness, dysentery, toxoplasmosis, ringworm, candidiasis, histoplasmosis, ebola, Acinetobacter infections, Actinomycosis, African sleeping sickness (African trypanosomiasis), AIDS (Acquired immunodeficiency syndrome), HIV infection, Amebiasis, Anaplasmosis, Anthrax, Arcanobacterium haemolyticum infection, Argentine hemorrhagic fever, Ascariasis, Aspergillosis, Astrovirus infection, Babesiosis, Bacillus cereus infection, Bacterial pneumonia, Bacterial vaginosis (BV), Bacteroides infection, Balantidiasis, Baylisascaris infection, BK virus infection, Black piedra, Blastocystis hominis infection, Blastomycosis, Bolivian hemorrhagic fever, Borrelia infection, Botulism (and Infant botulism), Brazilian hemorrhagic fever, Brucellosis, Bubonic plague, Burkholderia infection, Buruli ulcer, Calicivirus infection (Norovirus and Sapovirus), Campylobacteriosis, Candidiasis (Moniliasis; Thrush), Cat-scratch disease, Cellulitis, Chagas Disease (American trypanosomiasis), Chancroid, Chickenpox, Chikungunya, Chlamydia, Chlamydophila pneumoniae infection (Taiwan acute respiratory agent or TWAR), Cholera, Chromoblastomycosis, Clonorchiasis, Clostridium difficile infection, Coccidioidomycosis, Colorado tick fever (CTF), Common cold (Acute viral rhinopharyngitis; Acute coryza), Creutzfeldt-Jakob disease (CJD), Crimean-Congo hemorrhagic fever (CCHF), Cryptococcosis, Cryptosporidiosis, Cutaneous larva migrans (CLM), Cyclosporiasis, Cysticercosis, Cytomegalovirus infection, Dengue fever, Dientamoebiasis, Diphtheria, Diphyllobothriasis, Dracunculiasis, Ebola hemorrhagic fever, Echinococcosis, Ehrlichiosis, Enterobiasis (Pinworm infection), Enterococcus infection, Enterovirus infection, Epidemic typhus, Erythema infectiosum (Fifth disease), Exanthem subitum (Sixth disease), Fasciolopsiasis, Fasciolosis, Filariasis, Food poisoning by Clostridium perfringens, Free-living amebic infection, Fusobacterium infection, Gas gangrene (Clostridial myonecrosis), Geotrichosis, Gerstmann-Sträussler-Scheinker syndrome (GSS), Giardiasis, Glanders, Gnathostomiasis, Gonorrhea, Granuloma inguinale (Donovanosis), Group A streptococcal infection, Group B streptococcal infection, Haemophilus influenzae infection, Hand, foot and mouth disease (HFMD), Hantavirus Pulmonary Syndrome (HPS), Heartland virus disease, Helicobacter pylori infection, Hemolytic-uremic syndrome (HUS), Hemorrhagic fever with renal syndrome (HFRS), Hepatitis A, Hepatitis B, Hepatitis C, Hepatitis D, Hepatitis E, Herpes simplex, Histoplasmosis, Hookworm infection, Human bocavirus infection, Human ewingii ehrlichiosis, Human granulocytic anaplasmosis (HGA), Human metapneumovirus infection, Human monocytic ehrlichiosis, Human papillomavirus (HPV) infection, Human parainfluenza virus infection, Hymenolepiasis, Epstein-Barr Virus Infectious Mononucleosis (Mono), Influenza (flu), Isosporiasis, Kawasaki disease, Keratitis, Kingella kingae infection, Kuru, Lassa fever, Legionellosis (Legionnaires' disease), Legionellosis (Pontiac fever), Leishmaniasis, Leprosy, Leptospirosis, Listeriosis, Lyme disease (Lyme borreliosis), Lymphatic filariasis (Elephantiasis), Lymphocytic choriomeningitis, Malaria, Marburg hemorrhagic fever (MHF), Measles, Middle East respiratory syndrome (MERS), Melioidosis (Whitmore's disease), Meningitis, Meningococcal disease, Metagonimiasis, Microsporidiosis, Molluscum contagiosum (MC), Monkeypox, Mumps, Murine typhus (Endemic typhus), Mycoplasma pneumonia, Mycetoma, Myiasis, Neonatal conjunctivitis (Ophthalmia neonatorum), (New) Variant Creutzfeldt-Jakob disease (vCJD, nvCJD), Nocardiosis, Onchocerciasis (River blindness), Paracoccidioidomycosis (South American blastomycosis), Paragonimiasis, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Plague, Pneumococcal infection, Pneumocystis pneumonia (PCP), Pneumonia, Poliomyelitis, Prevotella infection, Primary amoebic meningoencephalitis (PAM), Progressive multifocal leukoencephalopathy, Psittacosis, Q fever, Rabies, Respiratory syncytial virus infection, Rhinosporidiosis, Rhinovirus infection, Rickettsial infection, Rickettsialpox, Rift Valley fever (RVF), Rocky Mountain spotted fever (RMSF), Rotavirus infection, Rubella, Salmonellosis, SARS (Severe Acute Respiratory Syndrome), Scabies, Schistosomiasis, Sepsis, Shigellosis (Bacillary dysentery), Shingles (Herpes zoster), Smallpox (Variola), Sporotrichosis, Staphylococcal food poisoning, Staphylococcal infection, Strongyloidiasis, Subacute sclerosing panencephalitis, Syphilis, Taeniasis, Tetanus (Lockjaw), Tinea barbae (Barber's itch), Tinea capitis (Ringworm of the Scalp), Tinea corporis (Ringworm of the Body), Tinea cruris (Jock itch), Tinea manum (Ringworm of the Hand), Tinea nigra, Tinea pedis (Athlete's foot), Tinea unguium (Onychomycosis), Tinea versicolor (Pityriasis versicolor), Toxocariasis (Ocular Larva Migrans (OLM)), Toxocariasis (Visceral Larva Migrans (VLM)), Trachoma, Trinochccliasis, Trichinlosis, Trichomoniasis, Trichuriasis (Whipworm infection), Tuberculosis, Tularemia, Typhoid Fever, Ureaplasma urealyticum infection, Valley fever, Venezuelan equine encephalitis, Venezuelan hemorrhagic fever, Viral pneumonia, West Nile Fever, White piedra (Tinea blanca), Yersinia pseudotuberculosis infection, Yersiniosis, Yellow fever, and Zygomycosis.

In some cases, a method described herein comprises determining if an infection is active or latent. In some cases, gene expression quantification may provide a method for detecting, predicting, diagnosing, or monitoring an active infection. In some cases, a method described herein comprises detecting an active infection. In some cases, gene expression may be quantified through detection or sequencing of a population of interest. In some cases, gene expression quantification may provide a method for detecting, predicting, diagnosing, or monitoring a latent infection. In some cases, a method described herein comprises detecting a latent infection.

The methods provided herein may be used to detect cancer, particularly in a subject that has such cancer, is at risk of having such cancer, or is otherwise suspected of having such cancer. Examples of cancers include but are not limited to: brain cancer, head and neck cancer, throat cancer, mouth cancer, breast cancer, bone cancer, blood cancer, leukemia, lymphoma, lung cancer, kidney cancer, pancreatic cancer, stomach cancer, colon cancer, rectal cancer, skin cancer, cancer of the reproductive tract, prostate cancer, etc. In some cases, the methods provided herein are particularly useful for detecting non-hematological cancers, such as cancer of a solid organ (e.g., lung cancer, breast cancer, pancreatic cancer, etc.)

The methods may also be useful for detecting any other types of diseases or conditions of the subject. Often, they are useful for detecting rare genetic variations; or nucleic acid sequences that make up only a very small portion of the total nucleic acid population in the sample.

The methods provided herein may enable the generation of sequencing data with high efficiency, high accuracy, and/or high sensitivity. The methods generally may have a very low false positive rate, e.g., a false positive rate of less than 5%, 4%, 3%, 2%, 1%, 0.1%, 0.05%, 0.01%.

The methods provided herein may be useful for accurately determining a portion of the microbiome of a sample. Contaminating nucleic acids, such as from environmental contamination, may interfere with accurate determination of the microbiome. The methods provides herein may be employed to remove the contaminating nucleic acids and to accurately determine the portion of the microbiome of the sample.

As used throughout the specification herein, the term "about" when referring to a number or a numerical range means that the number or numerical range referred to is an approximation within experimental variability (or within statistical experimental error), and the number or numerical range may vary from, for example, from 1% to 15% of the stated number or numerical range. In examples, the term "about" refers to ±10% of a stated number or value.

As used herein, the term "or" is used to refer to a nonexclusive or, such as "A or B" includes "A but not B," "B but not A," and "A and B," unless otherwise indicated.

Many aspects of this procedure are possible to modify. It may be possible to use just a local baseline or just a global baseline. A statistical model of between sample variations in the EC abundances that has a larger variance than predicted may be used by counting statistics alone. Finally, there may be other ways to normalize for variations in library size, including using abundances of common ECs (e.g., but organisms that are unlikely to be found in the host).

### Examples

### Example 1: Cleaning cell-free DNA extraction components using cleaned method

Cleaning procedure or cleaned method can be used to inactivate, remove, or avoid contaminating DNA from a commercial cell-free DNA extraction kit (Omega Bio-tek, Norcross, GA, USA). The cleaned method comprises washing or replacement of four of the components as follows:
a) Washing the DNA-binding magnetic beads and diluting contaminants by exchanging the bead buffer 5 times in succession with TE (Tris-EDTA buffer, Integrated DNA Technologies, Coralville, IA, USA).
b) Eliminating the carrier RNA in kit. Experiments have established that the RNA carrier was not needed.
c) Replacing the proteinase K in kit with proteinase from another vendor (Sigma-Aldrich, St. Louis, MO, USA) and reducing the amount 5 fold.
d) Replacing the elution buffer in the kit with TE.

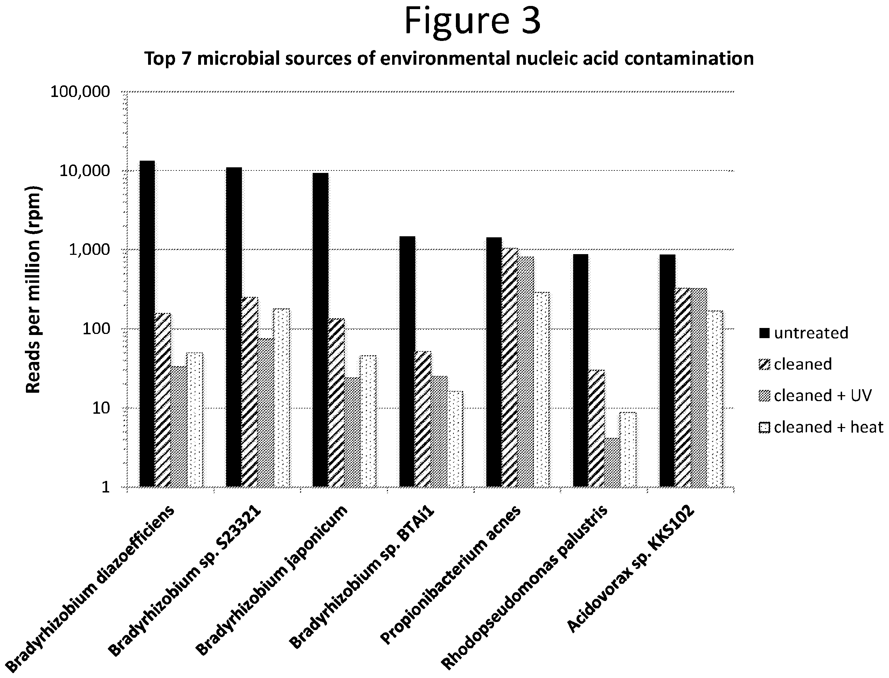

In one example, the effect of the cleaned method by employing steps (1) - (4) as described above was tested. A known quantity of synthetic DNA spikes was added to TE buffer followed by processing of these mock samples either with the standard extraction method (untreated) or with the cleaned method. The extracted DNA was then processed for sequencing by preparing NGS libraries. Sequencing was followed by identification of microbes represented by non-spiked DNA fragments. The non-spiked DNA fragments originated from environmental contaminants (EC). As shown in **Fig. 3****,** a reduction in the proportion of contaminating DNA or EC in the samples treated with the cleaned method when compared to untreated samples was observed. **Fig. 3** shows seven commonly contaminating microbes on the X-axis that are derived from the environment. The abundance of each of the contaminating microbes is shown on the Y-axis as indicated by reads per million (rpm). The reads per million provides a proportion of reads that uniquely map to a sequence of a microbe among all the reads to obtain normalized reads. The normalized reads are then multiplied by 1 million to obtain reads per million (rpm). The cleaned method alone, as shown by striped bars, is effective in reducing abundance of the contaminating microbes when compared to untreated samples as shown by black solid bars. The cleaned method alone is effective in reducing abundance of some taxa such as Bradyhrizobium and Rhodopseudomonas by approximately 90-99%. Other species were reduced to a lesser degree, indicating either that they were recalcitrant to the cleaning method or were introduced at other steps in the extraction/library/sequencing process.

### Example 2: UV treatment of DNA extraction reagents

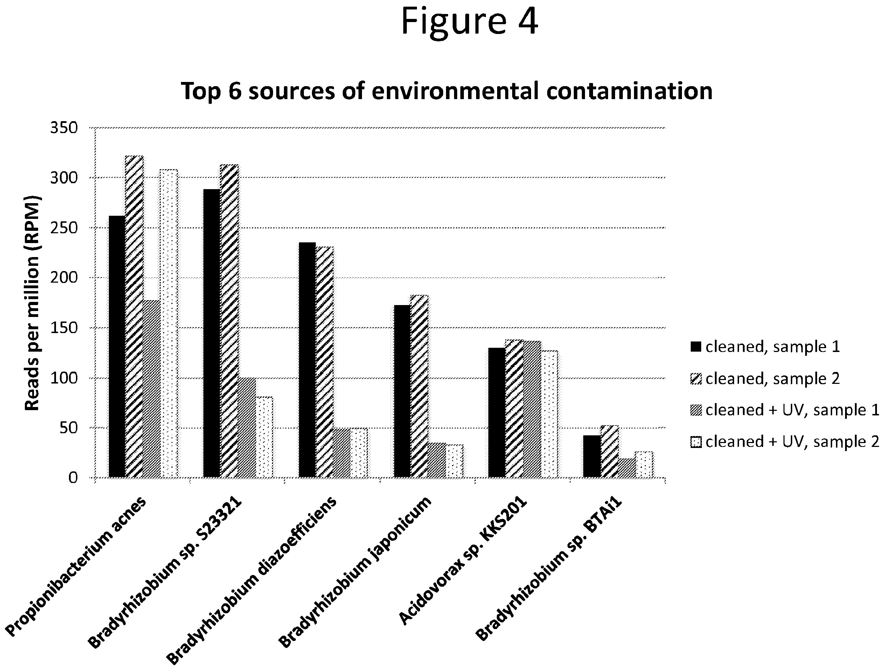

In addition to the steps employed in the cleaned method, additional buffers used for DNA extraction (e.g., lysis buffer, neutralization buffer, cleaned DNA-binding magnetic beads, wash buffers, and water) were treated with UV at a dose of 10,000 J/m² of 254 nm UV (Spectrolinker, Spectronics, Westbury, NY, USA) to reduce contaminating DNA from extraction reagents to produce the cleaned + UV samples. For comparison, the control samples were also prepared by subjecting the additional buffers to the cleaned method alone to produce the cleaned samples. The NGS libraries were made as described above using the cleaned + UV samples as well as using the cleaned samples. For example, as shown in **Fig. 4****,** the cleaned + UV sample (grey bars) demonstrated further reduction in the abundance of contaminating DNA when compared with the samples treated with cleaned method alone (striped bars). The further reduction for cleaned + UV when compared with the cleaned method alone was 50-80% for 5 of the 7 top taxa (4 Bradyrhizobium and Rhodopseudomonas contaminants), whereas 2 others were less affected (P. acnes and Acidovorax), suggesting that the latter 2 can be contaminants in reagents other than the buffers treated with UV. The cleaned method and cleaning + UV treatments were repeated in an independent experiment with 2 samples per condition. In an independent experiment, two technical replicates were included per treatment. For example, as shown in **Fig. 4****,** cleaned sample 1 and cleaned sample 2 are replicates of the control samples, prepared using the cleaned method alone. Similarly, cleaned + UV sample 1 and cleaned + UV sample 2 are replicates for the cleaned + UV samples, prepared using the cleaned method as well as the UV treatment. As shown in **Fig. 4****,** the cleaned + UV sample 1 and sample 2, as represented respectively by grey and stippled bars, showed further reduction in the abundance of contaminating microbes when compared with the samples treated with cleaned method alone, as indicated by black and striped bars representing cleaned sample 1 and cleaned sample 2, respectively. Four of the 6 top contaminating DNA species were reduced 50-80%, whereas P. acnes and Acidovorax tax were again reduced at a very low level or inconsistently.

### Example 3: Heat treatment of DNA extraction reagents

In addition to the steps employed in the cleaned method, additional buffers used for DNA extraction (lysis buffer, neutralization buffer, cleaned DNA-binding magnetic beads, wash buffers, and water) were treated with heat. In this example, the DNA-binding magnetic beads were incubated at 95 degrees Celsius for 5 hours with agitation to keep the beads in suspension, and the other reagents were placed in an incubator oven at 95 degrees Celsius for 5 hrs to reduce contaminating DNA from the DNA extraction reagents. Immediately after heating, reagents were cooled rapidly to approximately room temperature by moving them to an ice bath. NGS libraries were made as described above using heat-treated DNA extraction reagents (cleaned + heat). As shown in **Fig. 3****,** the cleaned + heat samples, as indicated by stippled bars, showed reduction in abundance of contaminating microbes when compared with untreated samples. The cleaned+ heat samples showed reduction in abundance of all seven when compared with samples treated with cleaned method alone. The cleaned +heat samples showed similar abundance when compared to cleaned + UV, with some taxa showing slightly higher reduction (up to 2X) for one treatment and the others slightly higher reduction (up to 2X) for the other treatment..

### Example 4: UV treatment of control nucleic acid libraries

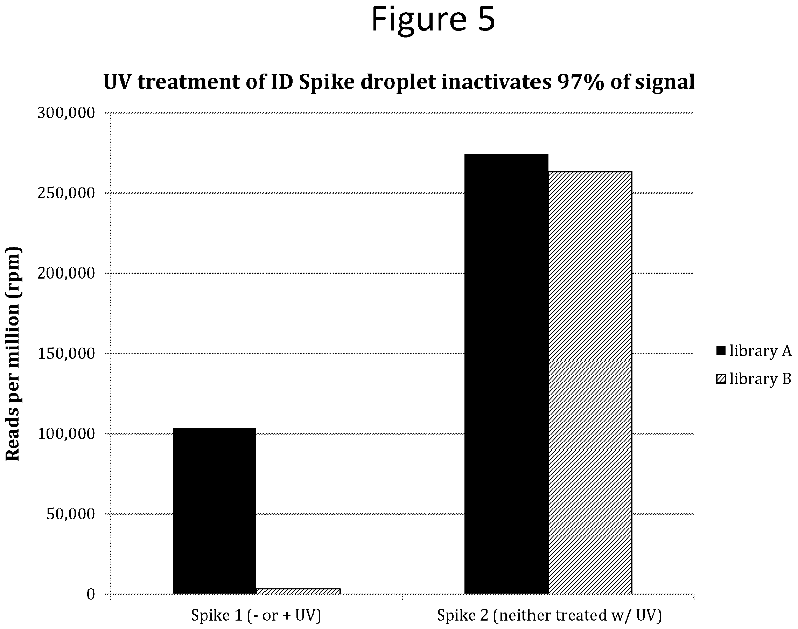

In a control experiment, two libraries (Library A and Library B) were prepared using two different synthetic DNAs (Spike 1 and Spike 2) in an aqueous buffer. Spike 1 and Spike 2 were not subjected to an extraction procedure but were rather treated in small droplets of 20 uL. Spike 1 was treated with UV (UV-treated DNA) before preparing Library A while Spike 1 was not treated with UV (non UV-treated DNA) before preparing Library B. Spike 2 was not treated with UV before preparing Library A and Library B. As shown in **Fig. 5****,** the UV-treated DNA of Library B lost 97% of the signal compared to a non-UV-treated DNA of Library A. The loss of signal in Library B of Spike 1was due to the UV treatment rather than a function of variations in library preparation since libraries produced by using Spike 2 gave a similar signal. This result demonstrates that the UV dose is sufficient to largely inactivate DNA if the UV thoroughly contacts the DNA in the reagents.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### ASPECTS OF THE INVENTION

1. A method for inactivating contaminant nucleic acids, the method comprising:
   (a) heating a nucleic acid analysis buffer, wherein the nucleic acid analysis buffer comprises contaminant nucleic acids and wherein the heating lasts for a length of time sufficient to inactivate at least a portion of the contaminant nucleic acids, thereby producing a decontaminated nucleic acid analysis buffer; and
   (b) contacting the decontaminated nucleic acid analysis buffer of step (a) with a sample comprising target nucleic acids.
2. A method of conducting a nucleic acid assay, the method comprising:
   (a) exposing a nucleic acid analysis buffer to non-ionizing radiation or to a nuclease, wherein the nucleic acid analysis buffer comprises contaminant nucleic acids and wherein the exposure to the non-ionizing radiation or to the nuclease lasts for a length of time sufficient to inactivate at least a portion of the contaminant nucleic acids, thereby producing a decontaminated nucleic acid analysis buffer;
   (b) contacting the decontaminated nucleic acid analysis buffer of step (a) with a sample comprising target nucleic acids; and
   (c) contacting the target nucleic acids in the sample with amplification or sequencing reagents, wherein the amplification or sequencing reagents have not been subjected to endonuclease treatment to remove contaminant nucleic acids.
3. A method for inactivating contaminant nucleic acids in a buffer, the method comprising:
   (a) obtaining a nucleic acid analysis buffer, wherein the nucleic acid analysis buffer comprises: (i) a reagent that is not water and (ii) contaminant nucleic acids; and
   (b) exposing the nucleic acid analysis buffer to ionizing radiation or a nuclease for a length of time sufficient to inactivate at least a portion of the contaminant nucleic acids, thereby producing a decontaminated nucleic acid analysis buffer.
4. A method of conducting a sequencing assay, the method comprising:
   (a) heating a nucleic acid analysis buffer, wherein the nucleic acid analysis buffer comprises contaminant nucleic acids and wherein the heating lasts for a length of time sufficient to inactivate at least a portion of the contaminant nucleic acids, thereby producing a decontaminated nucleic acid analysis buffer;
   (b) contacting the decontaminated nucleic acid analysis buffer of step (a) with a sample comprising target nucleic acids;
   (c) attaching double stranded adapters to the target nucleic acids in order to produce tagged target nucleic acids; and
   (d) subjecting the tagged target nucleic acids to a sequencing assay.
5. The method of aspect 4, further comprising performing an amplification reaction after attaching the double stranded adapters to the target nucleic acids.
6. The method of any one of aspects 1-5, wherein the contaminant nucleic acids are double-stranded nucleic acids.
7. The method of any one of aspects 1-6, wherein the contaminant nucleic acids are single stranded nucleic acids.
8. The method of any one of aspects 1-7, wherein the target nucleic acids are double-stranded nucleic acids.
9. The method of aspect 8, wherein the double-stranded target nucleic acids are natural double-stranded nucleic acids present in the sample prior to any sample processing.
10. The method of aspect 8, wherein the double-stranded target nucleic acids are double-stranded as a result of an *in vitro* amplification reaction.
11. The method of any one of the preceding aspects, wherein the contaminant nucleic acids comprise RNA or DNA.
12. The method of any one of aspects 1-11, wherein the target nucleic acids comprise RNA or DNA.
13. The method of any one of aspects 1-12, wherein the contaminant nucleic acids are less than 75 nucleotides in length.
14. The method of any one of aspects 1-13, wherein the contaminant nucleic acids are greater than 1,000 nucleotides in length.
15. The method of any one of aspects 1-14, wherein the contaminant nucleic acids are a mixture of nucleic acids having different lengths, wherein a portion of the contaminant nucleic acids contains short nucleic acids less than 75 nucleotides in length and a portion of the contaminant nucleic acids contains long nucleic acids greater than 500 nucleotides in length.
16. The method of aspect 15, wherein the short nucleic acids are inactivated by applying heat to the nucleic acid analysis buffer and the long nucleic acids are inactivated by applying non-ionizing radiation to the nucleic acid analysis buffer.
17. The method of any one of aspects 1-16, wherein the nucleic acid analysis buffer is rotated or agitated during the heating of the nucleic acid analysis buffer.
18. The method of any one of aspects 1-17, wherein the contaminant nucleic acids are derived from bacteria.
19. The methods of any one of aspects 1-18, wherein the contaminant nucleic acids comprise nucleic acids derived from soil bacteria, *Propionibacterium acnes, Bradyrhizobium* sp. S23321, *Bradyrhizobium diazoefficiens, Bradyrhizobium japonicum, Acidovorax* sp. KKS102, *Bradyrhizobium* sp. BTAi1, *Acidovorax temperans, Delftia sp. Csl-4, Delftia acidovorans, Methylobacterium acidovorans, Rhodopseudomonas palustris, Serratia marcescens, Acinetobacter junii, Acinetobacter johnsonii, Chryseobacterium indologenes* or any combination thereof.
20. The method of any one of the aspects 1-19, wherein the contaminant nucleic acids comprise nucleic acids derived from *Bradyrhizobium, Rhizobium*/*Agrobacterium, Sphingomonas, Burkholderia, Ralstonia, Pseudomonas, Stenotrophomonas, Flavobacterium, Bradyrhizobium sp. DFCI-1, Escherichia coli, Bos, Sus, Gallus, Herbaspirillum, Methylobacteria,* or any combination thereof.
21. The method of any one of the preceding aspects, wherein the contaminant nucleic acids are present at a concentration of at least 0.1% of total nucleic acids prior to inactivation.
22. The method of any one of the preceding aspects, wherein the contaminant nucleic acids are present at a concentration of up to 0.001% after inactivation.
23. The method of any one of the preceding aspects, wherein a signal from the contaminant nucleic acids is reduced by at least 50% following the inactivation.
24. The method of any one of the preceding aspects, wherein the inactivating the contaminant nucleic acids prevents the contaminant nucleic acids from being sequenced or PCR amplified.
25. The method of any one of the preceding aspects, wherein the inactivating the contaminant nucleic acids denatures the contaminant nucleic acids.
26. The method of aspect 2 or 3, wherein the inactivating the contaminant nucleic acids generates pyrimidine dimers in the contaminant nucleic acids.
27. The method of aspect 26, wherein the pyrimidine dimers are selected from cyclobutane pyrimidine dimers, thymine dimers, 6,4-photoproducts, and any combination thereof.
28. The method of aspect 2 or 3 or 4, wherein the inactivating the contaminant nucleic acids generates double-strand breaks or single-strand breaks in the contaminant nucleic acids.
29. The method of any one of the preceding aspects, wherein the nucleic acid analysis buffer is at least one buffer selected from the group consisting of a nucleic acid elution buffer, nucleic acid binding buffer, nucleic acid wash buffer, nucleic acid extraction buffer, hybridization reagent, equilibration buffer, purification reagent, sample preparation reagent, deparaffinization solution, cell lysis buffer, sample lysis buffer, tissue lysis buffer, viral particle lysis buffer, suspension or resuspension buffer, neutralization buffer, digestion buffer, equilibration buffer, solubilization buffer, blood/plasma stabilization reagent, blood component separation gel, and a PCR buffer.
30. The method of aspect 29, the nucleic acid binding buffer comprises nucleic acid binding particles.
31. The method of aspect 30, the nucleic acid binding particles comprise magnetic particles.
32. The method of any one of the preceding aspects, wherein the nucleic acid analysis buffer is an extraction buffer.
33. The method of aspect any one of the preceding aspects, further comprising extracting the target nucleic acids from the sample following the contacting of the nucleic acid analysis buffer with the sample comprising target nucleic acids.
34. The method of any one of the preceding aspects, wherein the nucleic acid analysis buffer comprises a detergent, a surfactant, a salt, a sugar, a protein, an alcohol, or a stabilizer.
35. The method of any one of the preceding aspects, wherein the nucleic acid analysis buffer comprises at least one detergent.
36. The method of any one of the preceding aspects, wherein the nucleic acid analysis buffer comprises glycerol.
37. The method of any one of the preceding aspects, wherein the nucleic acid analysis buffer is not a polymerase chain reaction (PCR) buffer or a ligase buffer.
38. The method of any one of the preceding aspects, further comprising amplifying a portion of the target nucleic acids with amplification reagents.
39. The method of aspect 1, wherein the nucleic acid analysis buffer retains at least 50% of its activity following the heating in step (a).
40. The method of any one of the preceding aspects, further comprising sequencing at least a portion of the target nucleic acids.
41. The method of aspect 40, wherein the sequencing comprises next generation sequencing.
42. The method of any one of the preceding aspects, further comprising amplifying at least a portion of the target nucleic acids.
43. The method of aspect 2, further comprising heating the nucleic acid analysis buffer prior to the contacting of step b.
44. The method of aspect 3, further comprising heating the nucleic acid analysis buffer.
45. The method of aspect 1, further comprising subjecting the nucleic acid analysis buffer to non-ionizing radiation.
46. The method of any one of the preceding aspects, further comprising cooling the nucleic acid analysis buffer following the heating.
47. The method of aspect 46, wherein the nucleic acid analysis buffer is cooled to room temperature.
48. The method of aspect 3, wherein the ionizing radiation is gamma radiation.
49. The method of aspect 2, wherein the non-ionizing radiation is ultraviolet radiation.
50. The method of any one of the preceding aspects, wherein the heating occurs at a temperature of at least about 80° C.
51. The method of any one of the preceding aspects, wherein the heating the nucleic acid buffer is performed for at least 30 minutes.
52. The method of aspect 1, further comprising irradiating the contaminant nucleic acids.
53. The method of aspect 52, wherein the irradiating comprises UV or gamma radiation.
54. The method of aspect 53, wherein a dose of UV irradiation is at least about 10,000 Joules per meter squared (J/m²).
55. The method of aspect 53, wherein a dose of gamma radiation is at least about 15 kiloGray (kGy).
56. The method of aspect 2, wherein the irradiating is at a frequency of at least 200 nm.
57. The method of any one of the preceding aspects, wherein the irradiating occurs for at least one minute.
58. The method of aspect 3, further comprising contacting the decontaminated nucleic acid analysis buffer with a sample comprising target nucleic acids.
59. The method of aspect 58, further comprising amplifying a portion of the target nucleic acids in the sample with amplification reagents.
60. The method of aspect 59, wherein the amplification reagents are not exposed to enzymatic treatment to degrade contaminant nucleic acids.
61. The method of any one of the preceding aspects, wherein the sample comprising target nucleic acids comprises intact cells.
62. The method of any one of the preceding aspects, wherein the target nucleic acids in the sample are derived from blood, plasma, serum, saliva, bronchoalveolar lavage, nasal swab, lymph, cerebrospinal fluid or urine.
63. The method of any one of the preceding aspects, wherein the target nucleic acids in the sample comprise cell-free nucleic acids originally present as cell-free nucleic acids in a circulating bodily fluid or urine.
64. The method of any one of the preceding aspects, wherein the sample comprises human nucleic acids.
65. The method of aspect 64, wherein the human has a pathogenic infection, is at risk of having a pathogenic infection, or is suspected of having a pathogenic infection.
66. The method of aspect 65, wherein the pathogenic infection is a bacterial, viral, fungal or parasitic infection.
67. The method of aspect 66, wherein the pathogenic infection is a bacterial infection.
68. The method of any one of the preceding aspects, wherein the contaminant nucleic acids are derived from a pathogen.
69. The method of any one of the preceding aspects, wherein the target nucleic acids are bacterial nucleic acids and the contaminant nucleic acids are bacterial nucleic acids.
70. The method of any one of the preceding aspects, wherein the target nucleic acids are bacterial nucleic acids and the contaminant nucleic acids are from the same species of bacteria as that of the target nucleic acids.
71. The method of any one of aspects 1-70, further comprising detecting a condition of the sample.
72. The method of aspect 70, wherein the condition is a pathogenic infection.
73. The method of aspect 2 or 3, wherein the nuclease is an endonuclease, RNase, or DNase.
74. The method of aspect 2 or 3, wherein the nuclease is DNase.
75. The method of aspect 3, wherein the reagent that is not water is a reagent selected from the group consisting of a detergent, a surfactant, a salt, a sugar, a protein, an alcohol, and a stabilizer.
76. The method of aspect 3, wherein the nucleic acid analysis buffer further comprises water.
77. The method of aspect 3, wherein the nucleic acid analysis buffer comprises at least one detergent.
78. The method of aspect 3, wherein the reagent that is not water is glycerol.
79. The method of any one of aspects 1-78, further comprising attaching an adapter to the target nucleic acids.

## Claims

1. A method of increasing the accuracy and efficiency of a nucleic acid assay, the method comprising:
(a) reducing or eliminating the signal produced by contaminant DNA fragments present in a nucleic acid analysis buffer by contacting the nucleic acid analysis buffer with an inactivation agent, wherein inactivation agent inactivates the contaminant DNA fragments present in the nucleic acid analysis buffer, thereby producing a decontaminated nucleic acid analysis buffer;
(b) contacting the decontaminated nucleic acid analysis buffer of step (a) with a sample comprising target double-stranded DNA (dsDNA); and
(c) attaching double-stranded adapters to the target dsDNA.

2. The method of claim 1, wherein the contaminant DNA fragments comprise contaminant double-stranded DNA (dsDNA) fragments.

3. The method of any one of claims 1-2, wherein the inactivation agent is radiation; optionally, the inactivation agent is nonionizing radiation; or optionally, the inactivation agent is ionizing radiation.

4. The method of claim 3, wherein:
(a) the nucleic acid analysis buffer is subjected to the radiation at a high enough dose to break DNA backbones of the contaminant dsDNA fragments present in the nucleic acid analysis buffer and reduce the signal produced by the dsDNA fragments to below detection limits of extraction or library preparation methods;
(b) the inactivation agent is radiation and the nucleic acid analysis buffer is subjected to the radiation at a high enough dose to produce pyrimidine dimers in the contaminant dsDNA fragments present in the nucleic acid analysis buffer and reduce the signal produced by the dsDNA fragments to below detection limits of extraction or library preparation methods; or
(c) the inactivation agent is radiation and the nucleic acid analysis buffer is subjected to the radiation at a high enough dose to produce bulky DNA lesions in the contaminant dsDNA fragments present in the nucleic acid analysis buffer and reduce the signal produced by the dsDNA fragments to below detection limits of extraction or library preparation methods.

5. The method of claim 2, wherein the inactivation agent is applied to the nucleic acid analysis buffer with a high enough dose to denature the contaminant dsDNA fragments present in the nucleic acid analysis buffer and reduce the signal produced by the dsDNA fragments to below detection limits of extraction or library preparation methods.

6. The method of any one of claims 1-7, wherein the nucleic acid assay is a sequencing assay wherein attaching double-stranded adapters to the target dsDNA produces tagged target nucleic acids, further comprising subjecting the tagged target nucleic acids to a sequencing assay.

7. The method of any one of claims 1-6, wherein the nucleic acid assay is a sequencing assay further comprising performing an amplification reaction after attaching the double-stranded adapters to the target dsDNA

8. The method of any one of the preceding claims, wherein the target dsDNA are selected from the group comprising double-stranded nucleic acids, natural double-stranded nucleic acids present in the sample prior to any sample processing, double-stranded target nucleic acids that are produced by an *in vitro* amplification reaction.

9. The method of any one of the preceding claims, wherein
(a) the contaminant DNA fragments are less than 75 nucleotides in length, greater than 1,000 nucleotides in length, or a mixture thereof; and/or
(b) the contaminant DNA fragments are present at a concentration of at least 0.1% of total nucleic acids prior to contacting the nucleic acid analysis buffer with the inactivation agent; and/or
(c) subsequent to contacting the nucleic acid analysis buffer with the inactivation agent, a signal from the contaminant DNA fragments is reduced by at least 50% or the contaminant DNA fragments are present at a concentration of up to 0.001%.

10. The method of any one of the preceding claims, wherein the nucleic acid analysis buffer is at least one buffer selected from the group consisting of a nucleic acid elution buffer, nucleic acid binding buffer, nucleic acid wash buffer, nucleic acid extraction buffer, hybridization reagent, equilibration buffer, purification reagent, sample preparation reagent, deparaffinization solution, cell lysis buffer, sample lysis buffer, tissue lysis buffer, viral particle lysis buffer, suspension or resuspension buffer, neutralization buffer, digestion buffer, equilibration buffer, solubilization buffer, blood/plasma stabilization reagent, blood component separation gel, a PCR buffer, and a sample preparation reagent comprising nucleic acid binding particles.

11. The method of any one of the preceding claims, wherein the nucleic acid analysis buffer is an extraction buffer; and/or comprises at least one of a detergent, surfactant, salt, sugar, protein, alcohol, glycerol, or stabilizer.

12. The method of any of claims 1-2, wherein the nucleic acid analysis buffer retains at least 50% of its activity following the inactivation in (a).

13. The method of any one of the preceding claims, wherein the sample comprising target dsDNA comprises intact cells, cell-free nucleic acids, cell-free nucleic acids originally present as cell-free nucleic acids in a circulating bodily fluid or urine, human nucleic acids, nucleic acids derived from blood, plasma, serum, saliva, bronchoalveolar lavage, nasal swab, lymph, cerebrospinal fluid or urine, and bacterial nucleic acids.

14. The method of claim 14, wherein the sample comprising target dsDNA is from a human wherein the human has a pathogenic infection, is at risk of having a pathogenic infection, or is suspected of having a pathogenic infection selected from the group of pathogenic infections consisting of bacterial, viral, fungal, or parasitic infections.

15. The method of any one of the preceding claims, wherein the contaminant nucleic acids are derived from a microbe.

16. The method of any one of the preceding claims, wherein the target dsDNA are bacterial nucleic acids and the contaminant nucleic acids are bacterial nucleic acids.

17. The method of any one of the preceding claims, wherein the nucleic acid analysis buffer comprises a reagent selected from the group consisting of a detergent, surfactant, salt, sugar, protein, alcohol, solvent, glycerol, and stabilizer.
